(19) **Europäisches Patentamt / European Patent Office / Office européen des brevets**

(11) **EP 2 737 842 B1**

(12) **EUROPEAN PATENT SPECIFICATION**

(45) Date of publication and mention of the grant of the patent:
**01.02.2017 Bulletin 2017/05**

(21) Application number: **12819613.6**

(22) Date of filing: **25.06.2012**

(51) Int Cl.:
*A61B 5/07* (2006.01)    *A61B 1/04* (2006.01)
*A61B 5/06* (2006.01)    *G01S 5/02* (2010.01)
*G01S 5/14* (2006.01)    *A61B 5/00* (2006.01)

(86) International application number:
**PCT/JP2012/066168**

(87) International publication number:
**WO 2013/018464 (07.02.2013 Gazette 2013/06)**

(54) **POSITION DETECTION APPARATUS, CAPSULE ENDOSCOPE SYSTEM, AND POSITION DETECTING PROGRAM FOR CAPSULE ENDOSCOPE**

POSITIONSERFASSUNGSGERÄT, KAPSELENDOSKOPSYSTEM UND POSITIONSERFASSUNGSPROGRAMM ZUM ERFASSEN EINER POSITION EINES KAPSELENDOSKOPS

APPAREIL DE DÉTECTION DE POSITION, SYSTÈME D'ENDOSCOPE DE TYPE CAPSULE ET PROGRAMME DE DÉTECTION DE POSITION POUR UN ENDOSCOPE DE TYPE CAPSULE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.07.2011   JP 2011167063**
**18.10.2011   JP 2011228556**

(43) Date of publication of application:
**04.06.2014   Bulletin 2014/23**

(73) Proprietor: **Olympus Corporation**
**Tokyo 192-8507 (JP)**

(72) Inventors:
• **HIGAKI, Naoya**
**Tokyo 151-0072 (JP)**

• **HOMAN, Masatoshi**
**Tokyo 151-0072 (JP)**
• **HASEGAWA, Jun**
**Tokyo 151-0072 (JP)**

(74) Representative: **Gunzelmann, Rainer**
**Wuesthoff & Wuesthoff**
**Patentanwälte PartG mbB**
**Schweigerstraße 2**
**81541 München (DE)**

(56) References cited:
**EP-A2- 1 260 176**    **JP-A- H08 500 441**
**JP-A- 2003 019 111**    **JP-A- 2005 514 969**
**JP-A- 2006 212 051**    **JP-A- 2006 212 051**
**JP-A- 2007 000 608**    **US-A1- 2002 193 685**
**US-A1- 2008 312 501**

**Description**

Field

The present invention relates to a position detection apparatus for detecting a position of a capsule endoscope inside a subject, a capsule endoscope system including the position detection apparatus, and a position detecting program for the capsule endoscope.

Background

In the field of the endoscopes in the related art, there is a known capsule endoscope in which an imaging function, a radio communication function, etc. are incorporated inside a capsule-shaped casing which is formed in a size that can be introduced into the digestive tract of a subject such as a patient. This capsule endoscope is swallowed from the mouth of the subject, and then moves inside the subject, such as the digestive tract, by peristalsis and the like. Further, the capsule endoscope sequentially captures images of the inside of the subject to generate image data, and sequentially radio-transmits the image data.

The image data thus radio-transmitted from the capsule endoscope is received by a receiving device provided outside the subject. The image data received by the receiving device is stored in a memory built inside the receiving device. After completion of examination, the image data stored in the memory of the receiving device is imported to an image display device. An observer, such as a doctor or a nurse, observes an organ image or the like displayed by the image display device, and diagnoses the subject.

Since the capsule endoscope moves inside the body cavity by peristalsis or the like, it is necessary to correctly recognize at which position in the body cavity the image data transmitted by the capsule endoscope is captured.

Considering above, disclosed is a capsule medical apparatus that receives electromagnetic wave transmitted from the capsule endoscope by a plurality of receiving antennas outside the body cavity, and estimates the position and orientation of the capsule endoscope by using a Gauss-Newton method based on reception strength of a plurality of received radio signals (see Patent Literature 1, for example).

Additionally, disclosed is a system for detecting a position of a capsule medical device, in which electromagnetic wave transmitted by the capsule endoscope is received by a plurality of receiving antennas outside the body cavity, an estimated region where the capsule endoscope is positioned is detected by each of the antennas based on the reception strength of a plurality of received radio signals, and an intersection of the respective regions is detected as the position of the capsule endoscope (see Patent Literature 2, for example).

Citation List

Patent Literature

Patent Literature 1: Japanese Laid-open Patent Publication No. 2007-000608
Patent Literature 2: Japanese Laid-open Patent Publication No. 2006-271987

EP 1 260 176 A1 concerns a system and method for localizing an in vivo signal source using a wearable antenna array having at least two antenna elements. The signal is received and a signal strength is measured at two or more antenna elements. An estimated coordinate set is derived from the signal strength measurements.

US 2008/312501 A1 concerns a capsule medical system that accurately detects the location at which biological information, such as an image captured in a living body, is acquired. A circular loop antenna incorporated in an endoscopic capsule traveling in a living body transmits a high-frequency signal and a plurality of antennas disposed on a body surface of the living body receives the signal. A CPU defines the initial location and orientation of the antenna. The CPU then performs an estimation process on the initial location and orientation so as to compute new estimated location and orientation and update the initial location and orientation to the new location and orientation. The CPU similarly performs the estimation process on the updated location and orientation. The CPU performs accurate location estimation by repeatedly performing the estimation process until the amount of shift of the location computed and updated by the estimation process reaches a sufficiently small value.

Summary

Technical Problem

However, when estimating the position and orientation of the capsule endoscope by performing the intricate calculation process such as the Gauss-Newton method like in Patent Literature 1, the position and orientation are calculated and estimated while being updated. Therefore, it is difficult to accelerate the position estimating process because of the large calculation amount.

In the position detecting system disclosed in Patent Literature 2, position detection accuracy for the capsule endoscope may be largely deteriorated because only the intersection of the respective regions is detected as the position of the capsule endoscope without estimating the orientation of the capsule endoscope.

The present invention has been made in view of the forgoing, and an object of the invention is to provide a position detection apparatus for the capsule endoscope that has constant accuracy while reducing the calculation amount, a capsule endoscope system including the position detection apparatus, and a position detecting program for the capsule endoscope.

Solution to Problem

In order to solve the above problem and achieve the object, the position detection apparatus according to claim 1 is provided.

According to the position detection apparatus of the invention, in the above invention, the detection unit is configured to determine whether a center point of each of a plurality of subregions is positioned inside the region where the at least three or more spheres are overlapped, the plurality of subregions being obtained by dividing a region inside the subject where the capsule endoscope possibly exists, and is configured to calculate the total sum of distances between the spherical surfaces and the center point of the subregion which has been determined to be positioned inside the region where the at least three or more spheres are overlapped, and is configured to detect, as the position of the capsule endoscope, the center point of the subregion where the total sum calculated by the detection unit is minimum. According to the position detection apparatus of the invention, in the above invention, the detection unit is configured to calculate, for each of the receiving antennas corresponding to the at least three or more spheres, differences between the first distance for each of the receiving antennas and a second distance which is a distance between each of the receiving antennas and the center point of the subregion which has been determined to be positioned inside the region where the at least three or more spheres are overlapped, and is configured to acquire a total sum of the calculated differences as the total sum of the distances between the center point of the subregion and the spherical surfaces.

According to the position detection apparatus of the invention, in the above invention, the detection unit is configured to correct the first distance based on dispersion of reception strength of each of the receiving antennas.

According to the position detection apparatus of the invention, in the above invention, the detection unit is configured to correct the first distance when the second distance which is a distance between the center point of the subregion and each of the receiving antennas is larger than the first distance for each of the receiving antennas.

According to the position detection apparatus of the invention, in the above invention, the detection unit is configured to determine whether the center point of each of the plurality of the subregions is positioned inside the region where all of the spheres corresponding to the plurality of the receiving antennas are overlapped.

In the above invention, the position detection apparatus according to the invention further includes a trajectory calculation unit configured to calculate a movement trajectory of the capsule endoscope based on the position of the capsule endoscope detected by the detection unit.

In the above invention, the position detection apparatus according to the invention further includes the plurality of the receiving antennas.

According to the position detection apparatus of the invention, in the above invention, the plurality of the receiving antennas are provided on a piece of sheet.

A capsule endoscope system according to the invention includes the features of claim 8.

According to the capsule endoscope system of the invention, in the above invention, the position detection apparatus further incudes a trajectory calculation unit configured to calculate a movement trajectory of the capsule endoscope based on the position of the capsule endoscope detected by the detection unit. The image display unit is configured to display the image information and also display the movement trajectory of the capsule endoscope inside the subject calculated by the trajectory calculation unit.

A position detecting program according to the invention including the features of claim 10.

According to the position detecting program of the invention, in the above invention, the detection procedure includes determining whether a center point of each of a plurality of subregions is positioned inside the region where the at least two or more spheres are overlapped, the plurality of subregions being obtained by dividing a region inside the subject where the capsule endoscope possibly exists, calculating the total sum of distances between the spherical surfaces and the center point of the subregion which has been determined to be positioned inside the region where the at least three or more spheres are overlapped, and detecting, as the position of the capsule endoscope, the center point of the subregion where the calculated total sum is minimum.

Advantageous Effects of Invention

According to the present invention, an orientation of the capsule endoscope is not detected, and thus the calculation amount is reduced. Further, a first distance between each of the plurality of receiving antennas and the capsule endoscope is calculated based on each reception strength of the signal received by the plurality of receiving antennas, a total sum of distances

from spherical surfaces is calculated inside a region where at least three or more spheres out of a plurality of spheres are overlapped, each of the plurality of spheres having a center at each of the plurality of receiving antennas and having a radius of the first distance. As a position of the capsule endoscope, a position where the total sum of distances from the spherical surfaces is minimum is detected. With this feature, it is possible to secure constant position detection accuracy.

Brief Description of Drawings

FIG. 1 is a schematic diagram illustrating a schematic configuration of a capsule endoscope system using a receiving device according to an embodiment of the present invention.

FIG. 2 is a cross-sectional view illustrating a schematic internal configuration of the capsule endoscope illustrated in FIG. 1.

FIG. 3 is a block diagram illustrating a schematic configuration of the receiving device illustrated in FIG. 1.

FIG. 4 is a flowchart illustrating a procedure related to a position detecting process in a position information estimating unit illustrated in FIG. 3.

FIG. 5 is a flowchart illustrating a procedure of position determining process illustrated in FIG. 4.

FIG. 6A is a schematic diagram for explaining detection of a capsule endoscope position.

FIG. 6B is a schematic diagram illustrating a region in FIG. 6A divided into quarters in each of x, y, and z directions.

FIG. 7 is a diagram illustrating a case where each of spheres centered at each of the receiving antennas and having a radius of a first distance corresponding to each of receiving antennas is taken along an xz plane.

FIG. 8 is a flowchart illustrating a procedure of a total distance calculation process illustrated in FIG. 5.

FIG. 9 is a diagram for explaining a calculation process of a capsule endoscope movement trajectory inside a subject executed by a trajectory calculation unit illustrated in FIG. 3.

FIG. 10 is a diagram for explaining the calculation process of the capsule endoscope movement trajectory inside the subject by the trajectory calculation unit illustrated in FIG. 3.

FIG. 11 is a flowchart illustrating another exemplary procedure of the total distance calculation process illustrated in FIG. 5.

FIG. 12 is a diagram for explaining still another exemplary procedure of the total distance calculation process illustrated in FIG. 5.

FIG. 13 is a diagram for explaining another different exemplary procedure of the total distance calculation process illustrated in FIG. 5.

FIG. 14 is a schematic diagram illustrating another schematic configuration of the capsule endoscope system using the receiving device according to an embodiment of the present invention.

FIG. 15 is a block diagram illustrating another configuration of the receiving device illustrated in FIG. 1.

Description of Embodiments

A position detection apparatus, a capsule endoscope system, and a position detecting program for the capsule endoscope according to an embodiment of the present invention will be described below by referring to the drawings. Note that a capsule endoscope system including a capsule endoscope which is introduced inside a subject to capture in-vivo images of the subject will be described below as an example of the position detection apparatus and the capsule endoscope system according to the present invention, but the present invention is not limited to the embodiment.

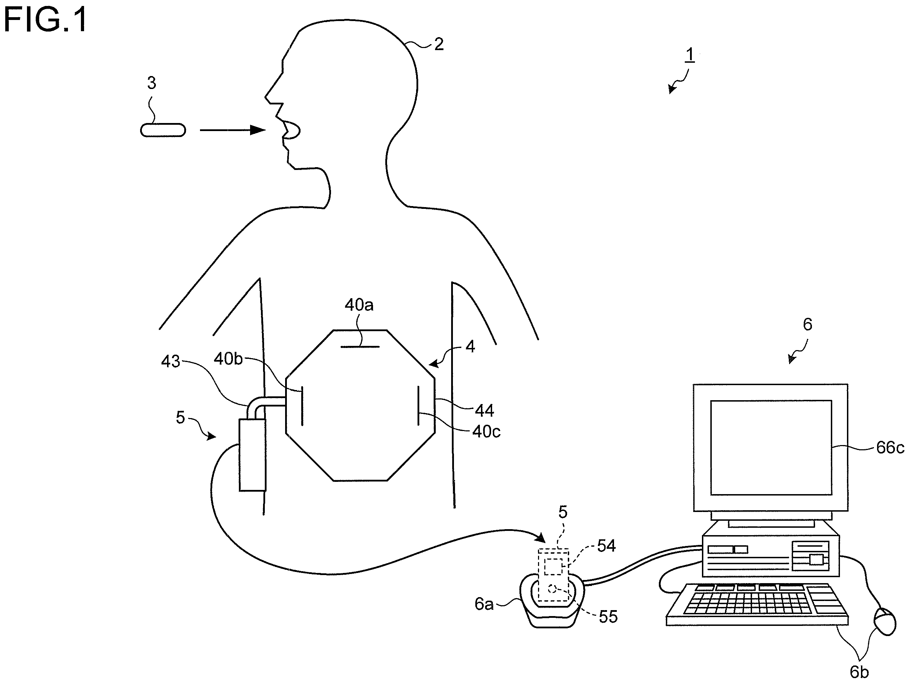

FIG. 1 is a schematic diagram illustrating a schematic configuration of a capsule endoscope system 1 using a receiving device 5 according to an embodiment of the present invention. As illustrated in FIG. 1, the capsule endoscope system 1 includes a capsule endoscope 3 that captures the in-vivo images of the inside of a subject 2, a receiving device 5 that receives a radio signal radio-transmitted by the capsule endoscope 3 introduced inside the subject 2 via a receiving antenna unit 4 while estimating an imaging position where image data of the inside of the subject 2 is captured by the capsule endoscope 3, and an information processor 6 that displays an image corresponding to the image data of the inside of the subject 2 captured by the capsule endoscope 3.

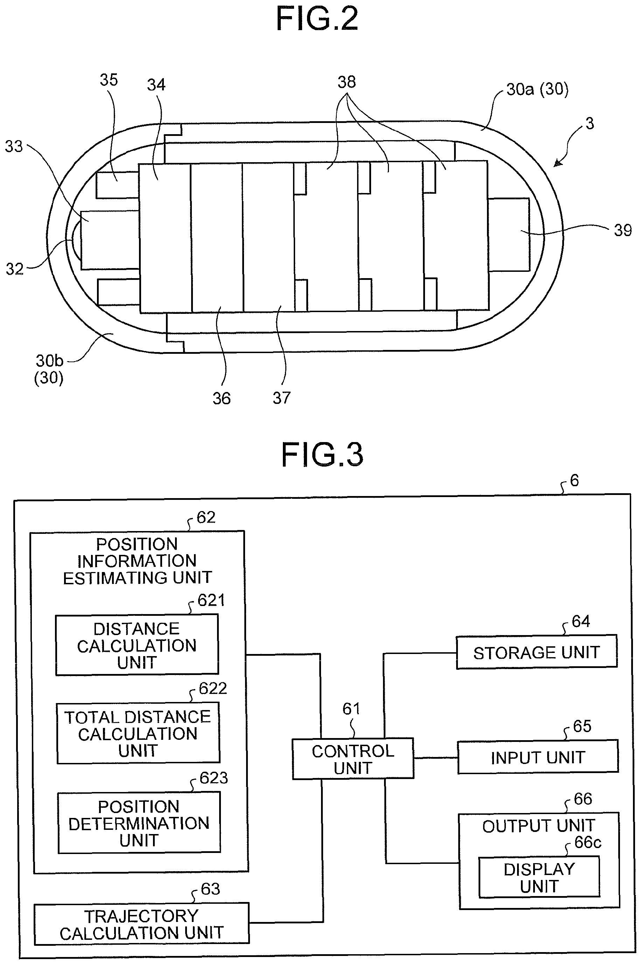

FIG. 2 is a cross-sectional view illustrating a schematic internal configuration of the capsule endoscope 3. As illustrated in FIG. 2, the capsule endoscope 3 is housed inside a capsule container 30 (casing) that includes: an approximately cylindrical or semi-ellipse sphere container 30a, one end of which is semi-sphere dome shape and the other end of which has an aperture; and an semi-sphere optical dome 30b fitted into the aperture of the container 30a to seal the container 30a water-tightly. The capsule container 30 (30a, 30b) is, for example, of a size that the subject 2 can swallow. Further, in the present embodiment, at least the optical dome 30b is formed of a transparent material.

Further, the capsule endoscope 3 includes an objective lens 32 that forms an image of the incident light through the optical dome 30b, a lens frame 33 by which the objective lens 32 is attached, an imaging unit 34 that converts an incident optical signal from the objective lens 32 to an electrical signal to form an imaging signal, a lighting unit 35 that lights the inside of the subject 2 at the time of imaging, a circuit board 36 on which a processing circuit and the like are formed to drive the imaging unit 34 and the lighting unit 35 respectively while generating an image signal based on the imaging signal input from the imaging unit 34, a transceiver circuit 37 that transmits the image signal and receives a signal from the receiving device 5 and the like provided outside the body cavity, a plurality of button batteries 38 that supplies power to each of function units, and an antenna 39.

The capsule endoscope 3 passes through the esophagus inside the subject 2 after being swallowed into the subject, and moves inside the body cavity by peristalsis of the digestive tract lumen. The capsule endoscope 3 successively captures images of the inside of the body cavity of the subject 2 at short time intervals, such as intervals of 0.5 seconds, while moving inside the body cavity, and generates the image data of the inside of the subject 2 which has been captured, and then sequentially transmits the image data to the receiving device 5. According to the present embodiment, it is possible to execute a position estimating process by the image signal of the image data captured by the imaging unit 34 of the capsule endoscope 3, but it is preferable to generate a transmission signal including the captured image signal and a reception strength detection signal for detecting a position of the capsule endoscope 3, and execute a position detecting process based on the reception strength detection signal by which the reception strength is easily detected.

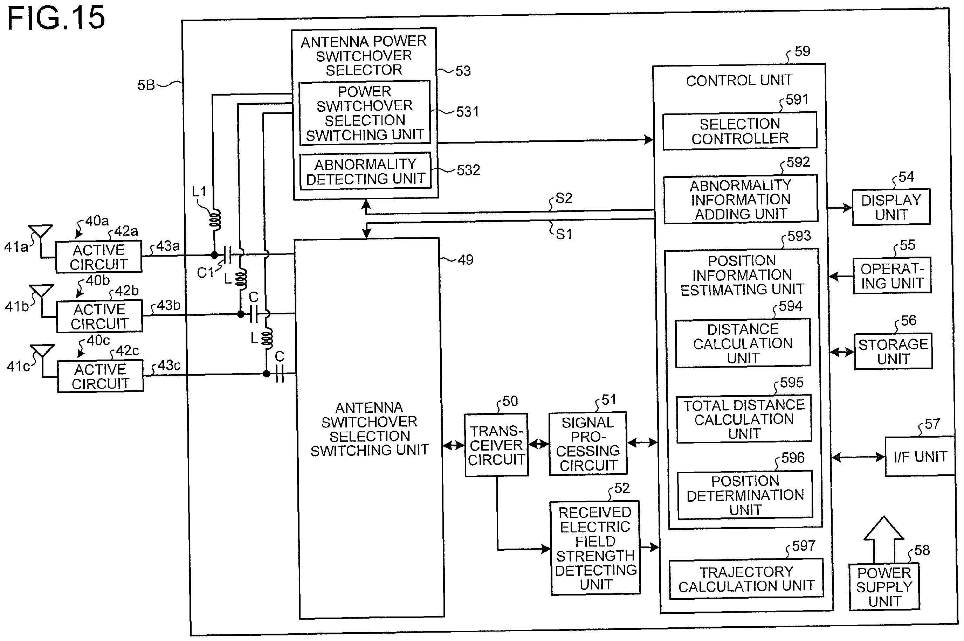

The receiving device 5 is connected, via an antenna cable 43, to the sheet-shaped receiving antenna unit 4 on which a plurality of receiving antennas 40 (40a, 40b, 40c) is arranged. The receiving device 5 receives a radio signal transmitted from the capsule endoscope 3 via each of a first receiving antenna 40a to a third receiving antenna 40c. The receiving device 5 detects received electric field strength of the radio signal received from the capsule endoscope 3 for each of the first receiving antenna 40a to the third receiving antenna 40c, and also obtains the image data of the inside of the subject 2 based on the received radio signal. The receiving device 5 correlates the received electric field strength information, time information indicating the time, etc. of each of the first receiving antenna 40a to the third receiving antenna 40c to the received image data, and stores these information in a storage unit (see FIG. 15) described later.

While the images are captured by the capsule endoscope 3, the receiving device 5 is carried by the subject 2 until the capsule endoscope 3 is excreted from the subject 2 after being introduced from the mouth of the subject 2 and passing through the digestive tract, for example. After completion of examination by the capsule endoscope 3, the receiving device 5 is detached from the subject 2 and connected to the information processor 6 in order to transmit the information such as the image data received from the capsule endoscope 3.

The first receiving antenna 40a to the third receiving antenna 40c are arranged at specified positions of a sheet 44, for example, at the positions corresponding to respective organs inside the subject 2 where the capsule endoscope 3 passes through when the receiving antenna unit 4 is attached to the subject 2. Note that the arrangement of the first receiving antenna 40a to the third receiving antenna 40c may be suitably changed depending on the purpose, such as examination or diagnosis. In the present embodiment, three receiving antennas are used, but the number of the receiving antennas is not limited to three and may be less or greater than three.

The information processor 6 is configured with a workstation or a personal computer including a display unit 66c such as a liquid crystal display. The information processor 6 displays the image corresponding to the image data of the inside of the subject 2 obtained via the receiving device 5. Further, the information processor 6 include a cradle 6a that reads the image data and the like from the storage unit of the receiving device 5, and an operation input device 6b such as a keyboard or a mouse. When the receiving device 5 is attached, the cradle 6a acquires image data from the memory of the receiving device 5 and related information, such as the received electric field strength information, time information, and identification information of the capsule endoscope 3 correlated with the image data, and transmits various acquired information to the information processor 6. The operation input device 6b accepts a user input. This allows the user to operate the operation input device 6b and observe living body sites of the subject 2, such as the esophagus, stomach, small intestine, and large intestine, and diagnose the subject 2 while watching the images of the inside of the subject 2 sequentially displayed by the information processor 6.

Next, the configuration of the information processor 6 illustrated in FIG. 1 will be described. FIG. 3 is a block diagram illustrating the configuration of the information processor 6 illustrated in FIG. 1.

As illustrated in FIG. 3, the information processor 6 is configured with the workstation or the personal computer that includes the display unit 66c such as the liquid crystal display. The information processor 6 displays the image corresponding to the image data of the inside of the subject 2 obtained via the receiving device 5. The cradle 6a that reads the image data from the memory of the receiving device 5, and the operation input device 6b such as the keyboard and the mouse are connected to the information processor 6.

Further, the information processor 6 includes, as illustrated in FIG. 3, a control unit 61 that controls the entire information processor 6, a position information estimating unit 62 that estimates position information of the capsule endoscope 3, a trajectory calculation unit 63 which calculates a movement trajectory of the capsule endoscope 3 inside the subject 2 based on the position information of the capsule endoscope 3 estimated by the position information estimating unit 62 for each of the image data, a storage unit 64 that stores the image data and the signal strength received from the capsule endoscope 3, an input unit 65 that obtains the information from the operation input device 6b and the like, such as the keyboard and mouse, the display unit 66c including the display, and an output unit 66 configured with a printer, a speaker and so on. Note that the storage unit 64 is configured with a hard disk that magnetically stores information, and a memory that electrically stores, by loading from the hard disk, various programs which are associated with the processes executed by the capsule endoscope system 1.

The position information estimation unit 62 acquires the maximum signal strength out of the signal strength received by each of the receiving antennas of the receiving antenna unit 4, and estimates the position of the capsule endoscope 3 by deriving the position information (the position of the antenna) of the capsule endoscope 3 from the signal strength. The position information estimating unit 62 includes a distance calculation unit 621, a total distance calculation unit 622, and a position determination unit 623.

The distance calculation unit 621 obtains a first distance which is the distance between the capsule endoscope 3 and each of the first receiving antenna 40a to the third receiving antenna 40c at the timing of position detecting based on each of the reception electric field strength received by each of the first receiving antenna 40a to the third receiving antenna 40c.

The total distance calculation unit 622 obtains a total sum of distances from respective spherical surfaces with respect to each of a specified unit area inside a region where at least three or more spheres out of a plurality of spheres are overlapped. Each of the plurality of the spheres centered at each of the receiving antennas has the radius of the first distance corresponding to each of the receiving antennas.

The position determination unit 623 determines, as a position of the capsule endoscope 3, a prescribed position of the unit area in which the total sum of the distances from the respective spherical surfaces is minimum among the total sums of the distances from the respective spherical surfaces in the respective unit areas, obtained by the total distance calculation unit 622.

According to the present embodiment, the information processor 6 includes the distance calculation unit 621, the total distance calculation unit 622, and the position determination unit 623, and the first distance which is the distance between the capsule endoscope 3 and each of the first receiving antenna 40a to the third receiving antenna 40c is obtained based on each of the reception strength of the signals received by the plurality of the first receiving antenna 40a to the third receiving antenna 40c. Then, the position detecting process is executed, in which the position in which the total sum of distances from the respective spherical surfaces is minimum in the region where at least two or more spheres out of the plurality of spheres are overlapped is determined as the position of the capsule endoscope 3. Each of the plurality of spheres centered at each of the first receiving antenna 40a to the third receiving antenna 40c has the radius of the first distance corresponding to each of the first receiving antenna 40a to the third receiving antenna 40c. In the following, the position detecting process for the capsule endoscope 3 in the information processor 6 according to the present embodiment will be described in detail.

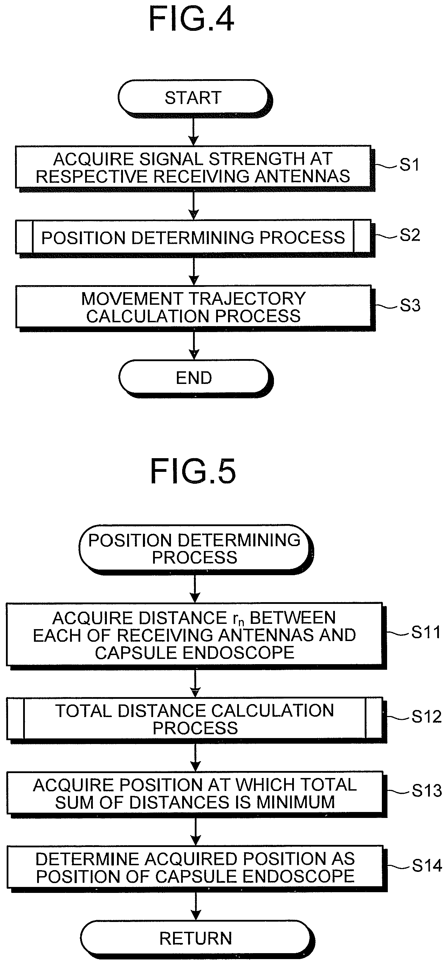

A procedure related to the position detecting process in the position information estimating unit 62 will be described in detail. FIG. 4 is a flowchart illustrating a procedure related to the position detecting process in the position information estimating unit 62 illustrated in FIG. 3.

As illustrated in FIG. 4, the position information estimating unit 62 acquires the signal strength of the received signal in each of the first receiving antenna 40a to the third receiving antenna 40c at the timing of position detection (step S1). Subsequently, the position information estimating unit 62 executes the position determining process to determine the position of the capsule endoscope 3 (step S2). After that, the trajectory calculation unit 63 executes the movement trajectory calculation process to calculate the movement trajectory of the capsule endoscope 3 inside the subject 2 based on the position information of the capsule endoscope 3 determined by the position information estimating unit 62 and the positions of the capsule endoscope 3 which have been detected so far (step S3).

Next, a procedure of the position determining process illustrated in FIG. 4 will be described. FIG. 5 is a flowchart illustrating the procedure of the position determining process illustrated in FIG. 4.

As illustrated in FIG. 5, the distance calculation unit 621 acquires, as the first distance, a distance $r_n$ between the capsule endoscope 3 and each of the first receiving antenna 40a to the third receiving antenna 40c based on the signal strength of the received signal in each of the first receiving antenna 40a to the third receiving antenna 40c (step S11). The distance calculation unit 621 obtains voltage $V_n$ of the received signal based on the received electric field strength of the received signal acquired for each of the first receiving antenna 40a to the third receiving antenna 40c, and acquires the distance $r_n$ between the capsule endoscope 3 and each of the first receiving antenna 40a to the third receiving antenna 40c using the following Expression (1), for each of the first receiving antenna 40a to the third receiving antenna 40c.

$$V_n = K \frac{1}{r_n} e^{-\alpha r_n}$$

$$(1)$$

In Expression (1), K represents a constant determined by characteristics of each of the first receiving antenna 40a to the third receiving antenna 40c, and $\alpha$ represents an attenuation coefficient of body tissue. In Expression (1), the constant K and the attenuation coefficient $\alpha$ can be derived from actual measurement values measured in advance. Since the constant K and the attenuation coefficient $\alpha$ vary in accordance with frequency, in the case where received frequency changes, the received frequency is estimated and calculated before executing the position determining process. In the case of estimating the received frequency, identification information including frequency information, product type, version information, etc. are stored in the storage unit of the receiving antenna unit 4, and the receiving device 5 acquires the identification information stored in the storage unit of the antenna unit 4, and then stores the identification information, correlating to the image data in the storage unit (not illustrated) inside the receiving device 5. At the time of calculating the constant K and the attenuation coefficient $\alpha$, the position information estimating unit 62 changes the received frequency in accordance with the identification information correlated to the image data. Additionally, in Expression (1), n represents an identification coefficient to identify the receiving antenna, and in the present embodiment, n is the value from 1 to 3 because the first receiving antenna 40a to the third receiving antenna 40c are set as the receiving antennas.

Subsequently, the total distance calculation unit 622 executes the total distance calculation process to obtain the total sum of distances from the respective spherical surfaces with respect to each of the specified unit area positioned inside a region where at least three or more spheres out of the plurality of spheres are overlapped (step S12). Each of the plurality of the spheres centered at each of the receiving antennas has the radius of the first distance corresponding to each of the receiving antennas.

The position determination unit 623 acquires a specified position of the unit area in which the total sum of distances from the respective spherical surfaces is minimum among the total sums of the distances from the respective spherical surfaces in the respective unit areas obtained by the total distance calculation unit 622 (step S13). Then, the acquired position is determined as the position of the capsule endoscope 3 (step S14). In the case where an image which has much noise and is not suitable to be displayed is stored, it is possible to add a process in which whether the image is to be displayed is determined, so that the position determining process is not executed for the image determined not to be displayed. In this case, the receiving device 5 or the information processor 6 detects the image which is not to be displayed, and adds non-display information (flag) to the image which is not to be displayed. This helps the position information estimating unit 62 to determine whether to execute the position determining process in accordance with the non-display information added to the image.

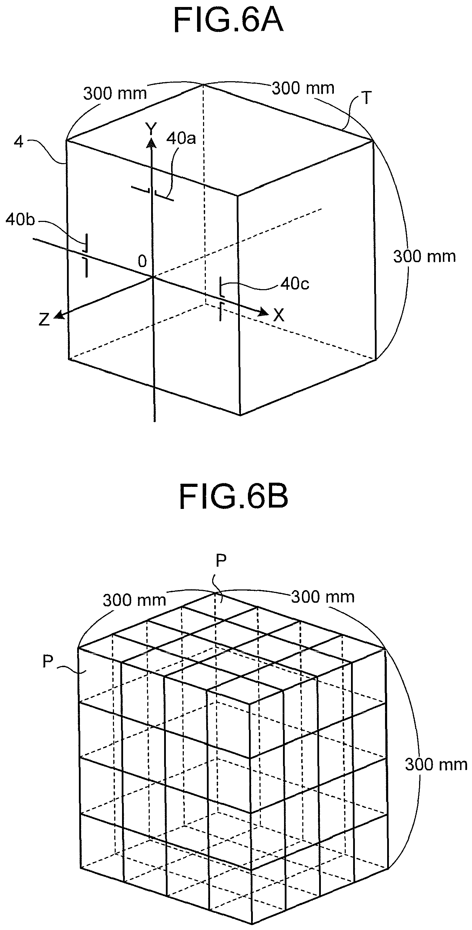

Next, the total distance calculation process executed by the total distance calculation unit 622 will be described. First, the specified unit area to be calculated in the total distance calculation process will be described. Primarily, a probable existence region T where the capsule endoscope 3 may exist is set inside the subject 2 into which the capsule endoscope 3 is introduced, in accordance with the purpose such as examination or diagnosis. This probable existence region T is set in accordance with the body size of the subject 2, and formed of, for example, a cube of 300 mm × 300 mm × 300 mm, as illustrated in FIG. 6A. The probable existence region T is set such that the sheet-shaped surface of the receiving antenna unit 4 coincides with one of boundary surfaces. In the case illustrated in FIG. 6A, the receiving antenna unit 4 is provided on the XY plane which is one of the boundary surfaces of the probable existence region T.

The probable existence region T of the capsule endoscope 3 is divided into a plurality of subregions in accordance with desired accuracy. For the sake of simplifying the description, an example is illustrated in FIG. 6B in which the center of the boundary surface on which the receiving antenna unit 4 is positioned is determined as an origin, and the probable existence region T is divided into four regions in each axis direction with respect to the orthogonal coordinate system XYZ having three axes (X axis, Y axis and Z axis) parallel to any one of the edges of the probable existence region T and orthogonal to one another. In this case, the probable existence region T is divided into 64 (= 4 × 4 × 4) subregions.

Each of the subregions is labeled so as to be identifiable. Each of the subregions P is determined as the specified unit area, and the total distance calculation process is executed as to the center of each of the subregions P. Note that actual subregions P are set in accordance with the size of the capsule endoscope 3 and the interval of the position detection timing, and the region is divided into, for example, 64 subregions as described above, or 27000 subregions each having the size of 10 mm $\times$ 10 mm $\times$ 10 mm.

The total distance calculation unit 622 determines, for each of the plurality of subregions P, whether the center point of each of the subregions P exists inside the region where the respective spheres are overlapped. The spheres centered at the respective receiving antennas have the radius of a distance $r_n$ which is the first distance corresponding to each of the receiving antennas. After that, the total distance calculation unit 622 obtains the total sum of the distances from the respective spherical surfaces to the center point of the subregion P that has been determined to exist inside the region where the respective spheres are overlapped.

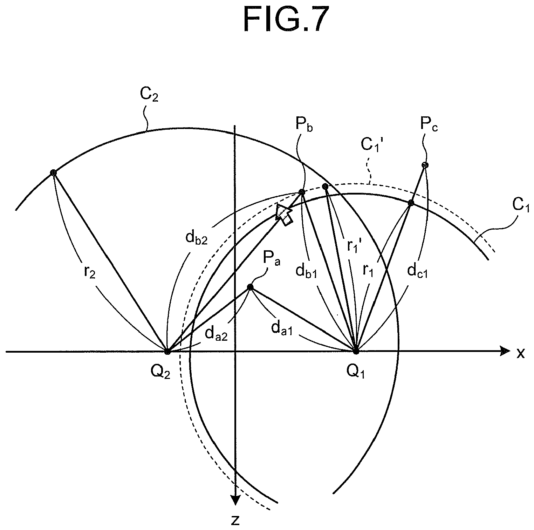

First, a concrete description will be given with reference to FIG. 7 regarding the determination process in which the total distance calculation unit 622 determines whether the center point of each of the subregions P is positioned inside the region where the respective spheres are overlapped. For the sake of simplifying the description, an example is illustrated in FIG. 7, in which each of the spheres centered at each of the receiving antennas and having the radius of the first distance $r_n$ corresponding to each of the receiving antennas, is taken along the xz plane. Additionally, in FIG. 7, a reference position $Q_1$ of the first receiving antenna 40a and a reference position $Q_2$ of the second receiving antenna 40b are illustrated.

The total distance calculation unit 622 first determines whether the center point of the subregion P to be determined is positioned inside the respective spheres $C_1$ and $C_2$ in order to determine whether the center point of each of the subregions P is positioned inside the region where the respective spheres are overlapped. In the case where that a first distance between the first receiving antenna 40a and the capsule endoscope 3 is set as $r_1$, the capsule endoscope 3 is estimated to be positioned at any position inside the sphere $C_1$ centered at the reference position $Q_1$ of the first receiving antenna 40a and having the radius $r_1$. Further, in the case where a first distance between the second receiving antenna 40b and the capsule endoscope 3 is set as $r_2$, the capsule endoscope 3 is estimated to be positioned at any position inside the sphere $C_2$ centered at the reference position $Q_2$ of the second receiving antenna 40b and having the radius $r_2$. Then, the total distance calculation unit 622 determines, for each of the receiving antennas, whether the center point of the subregion P to be determined is positioned inside the sphere corresponding to the receiving antenna by comparing a distance $d_{a1}$ between the center point of the subregion P to be determined and each of the receiving antennas to the first distance between the receiving antenna and the capsule endoscope 3.

For example, an exemplary case in which the center point of the subregion P to be determined is a point $P_a$ illustrated in FIG. 7 will be described. First, the total distance calculation unit 622 determines whether the point $P_a$ is positioned inside the sphere $C_1$ centered at the reference position $Q_1$ of the first receiving antenna 40a and having the radius $r_1$. Since the distance $d_{a1}$ between the point $P_a$ and the reference point $Q_1$ of the first receiving antenna 40a is shorter than the distance $r_1$ between the first receiving antenna 40a and the capsule endoscope 3, the total distance calculation unit 622 determines that the point $P_a$ is positioned inside the sphere $C_1$ centered at the reference position $Q_1$ of the first receiving antenna 40a and having the radius $r_1$. Next, the total distance calculation unit 622 determines whether the point $P_a$ is positioned inside the sphere $C_2$ centered at the reference position $Q_2$ of the second receiving antenna 40b and having the radius $r_2$. Since a distance $d_{a2}$ between the point $P_a$ and the reference point $Q_2$ of the second receiving antenna 40b is shorter than the distance $r_2$ between the second receiving antenna 40b and the capsule endoscope 3, the total distance calculation unit 622 determines that the point $P_a$ is positioned inside the sphere $C_2$ centered at the reference position $Q_2$ of the second receiving antenna 40b and having the radius of the distance $r_2$. Then, if the distance between the point $P_a$ and the reference point of the third receiving antenna 40c is shorter than the distance $r_3$ between the third receiving antenna 40c and the capsule endoscope 3, the total distance calculation unit 622 determines that the center point $P_a$ of this subregion is positioned inside the region where the respective spheres are overlapped. The respective spheres centered at the respective receiving antennas have the radius $r_n$ which is the first distance corresponding to each of the receiving antennas.

Next, another exemplary case in which the center point of the subregion P to be determined is a point $P_b$ illustrated in FIG. 7 will be described. The total distance calculation unit 622 determines whether the point $P_b$ is positioned inside the sphere $C_1$ centered at the reference position $Q_1$ of the first receiving antenna 40a and having the radius $r_1$. As illustrated in FIG. 7, a distance $d_{b1}$ between the point $P_b$ and the reference point $Q_1$ of the first receiving antenna 40a is longer than the distance $r_1$ between the first receiving antenna 40a and the capsule endoscope 3.

In this case, the total distance calculation unit 622 corrects the first distance $r_1$ corresponding to the first receiving antenna 40a based on dispersion of the reception strength of the first receiving antenna 40a so as to continue the calculation process, and compares the corrected first distance $r_1'$ with the distance $d_{b1}$. Note that the total distance calculation unit 622 may correct not only the first distance $r_1$ corresponding to the first receiving antenna 40a to be compared but also the first distance $r_n$ of all of the antennas including the first receiving antenna 40a based on the dispersion of the reception strength of each of the receiving antennas.

In the example illustrated in FIG. 7, the distance $d_{b1}$ is equal to or less than the corrected first distance $r_1$' at the point $P_b$. Therefore, the total distance calculation unit 622 determines that the point $P_b$ is positioned inside the sphere $C_1$' centered at the reference position $Q_1$ of the first receiving antenna 40a and having the radius of the corrected first distance $r_1$'. Further, the total distance calculation unit 622 determines whether the point $P_b$ is positioned inside the sphere $C_2$ centered at the reference position $Q_2$ of the second receiving antenna 40b and having the radius $r_2$. In this case, a distance $d_{b2}$ between the point $P_b$ and the reference point $Q_2$ of the second receiving antenna 40b is shorter than the distance $r_2$ between the second receiving antenna 40b and the capsule endoscope 3. Therefore, the total distance calculation unit 622 determines that the point $P_b$ is positioned inside the sphere $C_2$ centered at the reference position $Q_2$ of the second receiving antenna 40b and having the radius $r_2$. In the same manner, if the distance between the point $P_b$ and a reference point of the third receiving antenna 40c is shorter than the distance $r_3$ between the third receiving antenna 40c and the capsule endoscope 3, the total distance calculation unit 622 determines that the center point $P_b$ of the subregion is positioned inside the region where the respective spheres are overlapped. The respective spheres centered at the respective receiving antennas have the radius $r_n$ which is the first distance corresponding to each of the receiving antennas.

Additionally, another exemplary case in which the center point of the subregion P to be determined is a point $P_c$ illustrated in FIG. 7 will be described. The total distance calculation unit 622 determines whether the point $P_c$ is positioned inside the sphere $C_1$ centered at the reference position $Q_1$ of the first receiving antenna 40a and having the radius $r_1$. As illustrated in FIG. 7, a distance $d_{c1}$ between the point $P_c$ and the reference point $Q_1$ of the first receiving antenna 40a is longer than the distance $r_1$ between the first receiving antenna 40a and the capsule endoscope 3. Also, since the distance $d_{c1}$ is longer than the corrected first distance $r_1$', the total distance calculation unit 622 determines that the point $P_c$ is not positioned inside the sphere $C_1$' centered at the reference position $Q_1$ of the first receiving antenna 40a and having the radius of the corrected first distance $r_1$'. In other words, since the point $P_c$ is positioned neither inside the sphere $C_1$ which is one of the respective spheres nor inside the sphere $C_1$' after the correction is made, it can be determined that the point $P_c$ is not positioned inside the region where the respective spheres are overlapped, and then is excluded from an estimated position of the capsule endoscope 3. Here, the respective spheres centered at the respective receiving antennas have the radius $r_n$ which is the first distance corresponding to each of the receiving antennas.

Thus, if, for all of the receiving antennas, a second distance which is the distance between the center point of the subregion P to be determined and the receiving antenna is shorter than the distance $r_n$ corresponding to the receiving antenna with respect to each of the plurality of the subregions P obtained by dividing the region inside the subject 2 where the capsule endoscope 3 may exist, the total distance calculation unit 622 determines that the center point of the subregion P is positioned in the region where all of the spheres corresponding to the respective receiving antennas are overlapped. Further, in the case where the second distance d which is the distance between the center point of the subregion P to be determined and the receiving antenna is longer than the distance $r_n$ corresponding to the receiving antenna, the total distance calculation unit 622 corrects the distance $r_n$ based on the dispersion of reception strength of the receiving antenna so as to continue the correct calculation process. Further, the total distance calculation unit 622 obtains the total sum of the distances between the respective spherical surfaces and the center point of the subregion P that has been determined to be positioned inside the region where all of the spheres corresponding to the respective receiving antennas are overlapped.

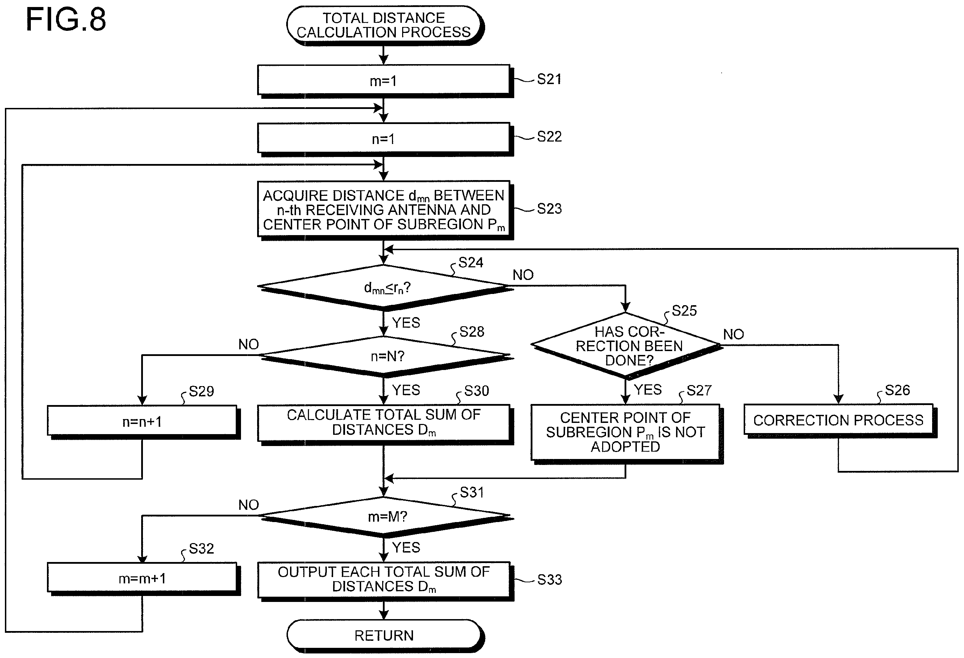

Next, a procedure of the total distance calculation process executed by the total distance calculation unit 622 will be described in detail. FIG. 8 is a flowchart illustrating the procedure of total distance calculation process illustrated in FIG. 5.

As illustrated in FIG. 8, the total distance calculation unit 622 first initializes an identification coefficient m of each of the subregions to be m = 1 (step S21), and executes the total distance calculation process for a subregion $P_1$ which is a target of the total distance calculation. The total distance calculation unit 622 initializes an identification coefficient n of the receiving antenna to be n = 1 in order to acquire a distance between the calculation target subregion $P_1$ and each of the receiving antennas (step S22). Subsequently, a distance $d_{mn}$ between an n-th receiving antenna (in this case, the first receiving antenna 40a) and the center point of a subregion $P_m$ (in this case, a subregion $P_1$) is acquired (step S23).

Next, the total distance calculation unit 622 compares the obtained distance $d_{mn}$ with the distance $r_n$ which is the first distance corresponding to the n-th receiving antenna, and determines whether the distance $d_{mn}$ is equal to or shorter than the distance $r_n$ which is the first distance corresponding to the n-th receiving antenna (step S24).

If the distance $d_{mn}$ is not equal to or not shorter than the distance $r_n$ corresponding to the n-th receiving antenna (step S24: No), in other words, in the case where the distance $d_{mn}$ is longer than the distance $r_n$ corresponding to the n-th receiving antenna, the total distance calculation unit 622 determines whether correction process has been executed in the current subregion $P_m$ (step S25).

If the correction process has not been executed in the current subregion $P_m$ (step S25: No), the total distance calculation unit 622 executes the correction process to correct the distance $r_n$ in response to dispersion of the reception strength of the n-th receiving antenna (step S26). After that, the total distance calculation unit 622 returns to step S24 and determines whether the distance $d_{mn}$ is equal to or shorter than the corrected distance $r_n$ by comparing the distance $d_{mn}$ with the corrected distance $r_n$.

On the other hand, if the correction process has been executed in the current subregion $P_m$ (step S25: Yes), the total distance calculation unit 622 may determine that the center point of this subregion $P_m$ is positioned farther than the capsule endoscope 3 from the n-th receiving antenna and is not positioned inside the sphere centered at the n-th receiving antenna and having the radius of the distance $r_n$. As a result, the subregion $P_m$ may be excluded from the region where the capsule endoscope 3 is expected to exist. Therefore, the total distance calculation unit 622 determines that the center point of the subregion $P_m$ is not adoptable as an estimated position of the capsule endoscope 3 (step S27), and excludes the subregion $P_m$ from the estimated position of the capsule endoscope 3.

Further, if the distance $d_{mn}$ is equal to or shorter than the distance $r_n$ (step S24: Yes), the total distance calculation unit 622 may determine that the center point of the subregion $P_m$ is positioned inside the sphere centered at the n-th receiving antenna and having the radius of the distance $r_n$. Subsequently, the total distance calculation unit 622 compares the identification coefficient n of each of the receiving antennas with a maximum value N of the identification coefficient of the receiving antenna, and determines whether n = N (step S28). If n is not equal to N (step S28: No), the total distance calculation unit 622 adds one to the identification coefficient n of the receiving antenna to be n = n + 1 (step S29), and determines for a next receiving antenna whether the center point of the subregion $P_m$ to be determined is positioned inside the sphere centered at the n-th receiving antenna and having the radius $r_n$. In this case, whether the center point of the subregion $P_m$ to be determined is positioned inside the sphere centered at the second receiving antenna and having the radius $r_2$ is determined.

In the case where the total distance calculation unit 622 determines that n is equal to N (step S28: Yes), it may be determined for all of the receiving antennas that the center point of the subregion $P_m$ to be determined is positioned inside the spheres centered at the respective receiving antennas and each having the radius of distance $r_n$. In other words, the center point of the subregion $P_m$ may be determined to be positioned inside the region where all of the spheres centered at the respective receiving antennas and each having the radius of the distance $r_n$ are overlapped.

For this reason, the total distance calculation unit 622 calculates a total sum of distances $D_m$ which is the total sum of distances ($r_n - d_{mn}$) between the center point of this subregion $P_m$ and each of the spherical surfaces with respect to n (step S30). More specifically, the total distance calculation unit 622 calculates the total sum of the distances $D_m$, using the following Expression (2).

$$D_m = \sum_{n=1}^{N} (r_n - d_{mn}) \tag{2}$$

Subsequently, the identification coefficient m of the subregion is compared with a maximum value M of the identification coefficient m of the subregion to determine whether m is equal to M (step S31). If m is not equal to M (step S31: No), the total distance calculation unit 622 adds one to the identification coefficient m of the subregion to be m = m + 1 (step S32). Then, returning to step S22, the total distance calculation unit 622 determines whether a next subregion $P_m$ is positioned inside the region where all of the spheres centered at each of the receiving antennas and each having a radius of the distance $r_n$ are overlapped. After that, the total sum of the distances $D_m$ which is the total sum of the distances between the center point of the subregion $P_m$ and each of the spherical surfaces is calculated. In this case, it is determined whether a next subregion $P_2$ is positioned inside the region where all of the spheres centered at each of the receiving antennas and each having a radius of the distance $r_n$ are overlapped.

Further, if m is equal to M (step S31: Yes), the total distance calculation unit 622 outputs each of the total sum of distances Dm for each of the subregions to the position determination unit 623 (step S33), thereby finishing total distance calculation process. In the case where the position of each of the receiving antennas changes depending on the type of the receiving antenna unit 4, the identification information including the frequency information, product type, version information, etc. are stored in the storage unit of the receiving antenna unit 4, and the receiving device 5 acquires the identification information stored in the storage unit of the antenna unit 4, correlating the identification information to the image data to store in the storage unit (not illustrated) inside the receiving device 5. The total distance calculation unit 622 may change a position parameter of a receiving antenna used for calculating the total sum of the distances, using the identification information correlated to the image data.

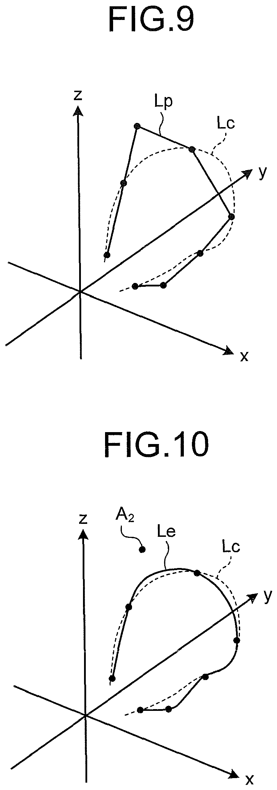

As described above, the position of the capsule endoscope 3 detected by the position information estimating unit 62 is sequentially stored in the storage unit 64, and used for the calculation of a movement trajectory of the capsule endoscope 3 inside the subject 2 executed by the trajectory calculation unit 63, together with the positions of the capsule endoscope 3 that have been detected so far. The detected position of the capsule endoscope 3 may include an error due to a positional error in each of the receiving antennas, noise, and so on. As illustrated in FIG. 9, a movement trajectory Lp of the capsule endoscope 3 obtained by adopting respective detected positions sometimes may differ from an actual movement trajectory Lc of the capsule endoscope 3. It can be considered that the capsule endoscope 3 may not largely move in a short time because the capsule endoscope 3 moves within an organ inside the subject 2.

Therefore, the trajectory calculation unit 63 calculates the trajectory, executing a correction process such as a median filtering process in which a median is obtained in temporally successive coordinates, for example, three coordinates including the coordinates temporally preceding and succeeding. As a result, as illustrated in FIG. 10, the trajectory calculation unit 63 may obtain the movement trajectory Lc closer to the actual movement trajectory Lp. This movement trajectory Lc is less influenced by a detecting position $A_2$ which is a position largely deviated from the actual movement trajectory Lp due to the positional error of each of the receiving antennas, noise, and so on. Additionally, not limited to the median filtering process, the trajectory calculation unit 63 may execute a movement averaging process in which the trajectory is calculated by obtaining an average value of five coordinates including, for example, two successive coordinates temporally preceding and succeeding. As a result, the trajectory calculation unit 63 may obtain the movement trajectory of the capsule endoscope 3 having reduced influence from the positional error of each of the receiving antennas, noise, and so on. Also, a low-pass filter process may be applied. Moreover, it is also possible to obtain the movement trajectory without time delay by executing the low-pass filter process temporally in both a forward direction and a reverse direction. The movement trajectory calculated is displayed together with the image data on the information processor 6.

According to the above-described embodiment of the present invention, only the position of the capsule endoscope is detected. Therefore, arithmetic process is simplified and a calculation amount is reduced compared to the case of obtaining both the position and orientation of the capsule endoscope, and as a result, the position estimating process can be accelerated.

Further, according to the present embodiment, the position where the total sum of the distances from the respective spherical surfaces is minimum is detected as the position of the capsule endoscope 3 in the region where all of the plurality of spheres is overlapped. The spheres have the radius of the first distance which is the distance between the capsule endoscope 3 and each of the receiving antennas obtained based on each of the reception strength of the signal received by each of the plurality of receiving antenna. It is clear that the capsule endoscope 3 is positioned inside the region where all of the plurality of spheres having the radius of the first distance which is the distance between the capsule endoscope 3 and each of the receiving antennas is overlapped. Further, according to the present embodiment, the position where the total sum of the distances from the respective spherical surfaces is minimum is estimated as the position of the capsule endoscope 3 inside the region where the plurality of spheres is overlapped. Accordingly, the constant position detection accuracy can be secured, and also the movement trajectory of the capsule endoscope 3 inside the subject 2 can be estimated more correctly.

Moreover, according to the present embodiment, it is not necessary to adjust the arrangement position of each of the receiving antennas 40 for every examination because the sheet-shaped receiving antenna unit 4 on which the plurality of receiving antennas 40 is arranged is used. Also, it is possible to avoid the problem of accuracy degradation in the estimation process for the capsule endoscope 3 position due to the positional deviation of each of the receiving antennas 40 because the receiving antenna unit 4 on which the arrangement position of each of the receiving antennas 40 is preliminarily determined is used.

Note that, according to the present embodiment, the total distance calculation unit 622 determines, as the total distance calculation process, whether the center point of the subregion P exists inside the region where the plurality of spheres centered at each of the receiving antennas and having the radius of the distance $r_n$ corresponding to each of the receiving antennas is overlapped, but the embodiment is not limited thereto. Additionally, even in the case where the center point of the subregion P does not exist inside the region where all of the spheres corresponding to all of the receiving antennas are overlapped, the total distance calculation unit 622 may proceed to the process to obtain the total sum of the distances from the respective spherical surfaces, instead of not adopting this center point, if the center point of the subregion P is positioned inside the region where the respective spheres corresponding to a specified number or more of the receiving antennas out of the plurality of receiving antennas are overlapped.

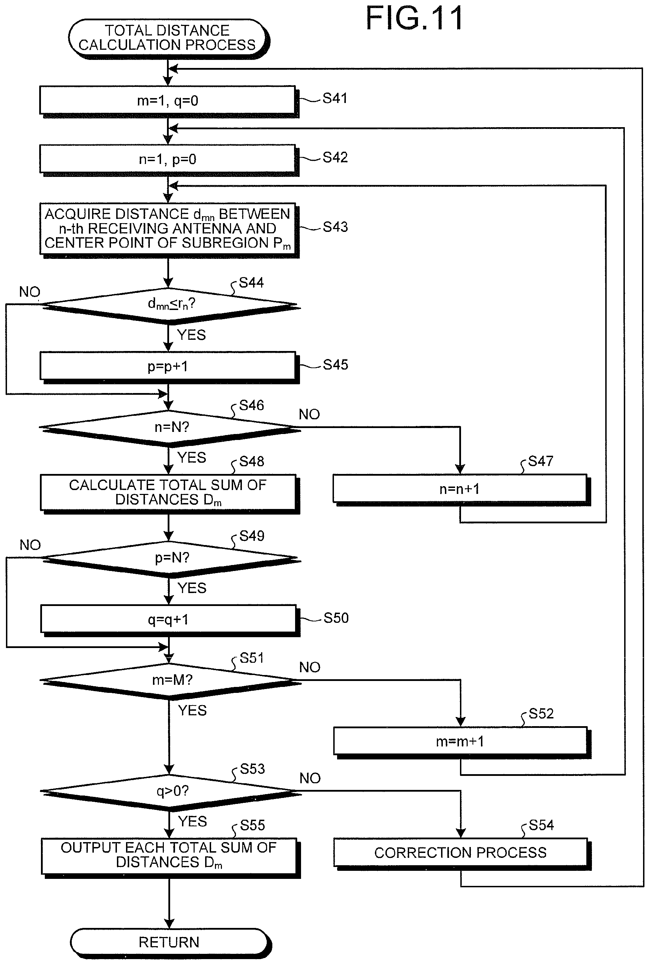

Further, in the total distance calculation process according to the present embodiment, the total sum of the distances is calculated for all of the subregions $P_m$. In the case where it is determined that the subregion is not positioned inside the region where any of the spheres are overlapped, it is possible to execute the correction process to correct the first distance in response to the dispersion of reception strength of all of the receiving antennas. A procedure of the total distance calculation process executed in this case will be described in detail. FIG. 11 is a flowchart illustrating another exemplary procedure of the total distance calculation process illustrated in FIG. 5.

First, as illustrated in FIG. 11, the total distance calculation unit 622 initializes the identification coefficient m of each of the subregions P to be m = 1, and also initializes q representing the number of the subregions P inside the region where all of hemispheres corresponding to each of the receiving antennas are overlapped, to be q = 0 (step S41). Then, the total distance calculation unit 622 executes the total distance calculation process for the subregion $P_1$ that is the target of the total distance calculation. The total distance calculation unit 622 initializes n representing the identification coefficient of the receiving antenna to be n = 1 in order to obtain the distances between the subregion $P_1$ to be calculated and the respective receiving antennas, and also initializes a coefficient p to be p = 0 (step S42). The coefficient p is to identify how many hemispheres are overlapped in the region where the subregion $P_n$ exists. For instance, in the case where p is three, the subregion $P_n$ is positioned in the region where three hemispheres corresponding to three receiving antennas are overlapped.

Next, the distance $d_{mn}$ between the n-th receiving antenna (in this case, the first receiving antenna 40a) and the center point of the subregion $P_m$ (in this case, the subregion $P_1$) is acquired (step S43).

Subsequently, the total distance calculation unit 622 compares the obtained distance $d_{mn}$ with the first distance $r_n$ corresponding to the n-th receiving antenna, and determines whether the distance $d_{mn}$ is equal to or shorter than the first distance $r_n$ corresponding to the n-th receiving antenna (step S44).

If the distance $d_{mn}$ is equal to or shorter than the distance $r_n$ (step S44: Yes), the total distance calculation unit 622 may determine that it is positioned inside the sphere centered at this n-th receiving antenna and having the radius of the distance $r_n$. Therefore, one is added to the coefficient p to be p = p + 1 to identify how many hemispheres are overlapped in the region where the subregion $P_n$ exists (step S45).

After completion of step S45, or in the case where it is determined that the distance $d_{mn}$ is not equal to or not shorter than the distance $r_n$ corresponding to the n-th receiving antenna (step S44: No), the total distance calculation unit 622 compares the identification coefficient n of the receiving antenna with the maximum value N of the identification coefficient of the receiving antenna, and determines whether n is equal to N (step S46). If n is not equal to N (step S46: No), the total distance calculation unit 622 adds one to the identification coefficient n of the receiving antenna to be n = n + 1 (step S47), and determines for a next receiving antenna whether the center point of the subregion $P_m$ to be determined is inside the sphere centered at the n-th receiving antenna and having the radius $r_n$. In this case, it is determined whether the center point of the subregion $P_m$ to be determined is positioned inside the sphere centered at the second receiving antenna and having the radius $r_2$.

In the case where the total distance calculation unit 622 determines n is equal to N (step S46: Yes), the same procedure in step S30 of FIG. 8 is executed to calculate the total sum of distances $D_m$ which is the total sum of the distances ($r_n$ - $d_{mn}$) between the center point of this subregion $P_m$ and the respective spherical surfaces with respect to n (step S48).

Subsequently, the total distance calculation unit 622 compares the maximum value N of the identification coefficient of the receiving antenna with the coefficient p which identifies how many hemispheres are overlapped in the region where the subregion $P_n$ exists, and determines whether p is equal to N (step S49). In the case where the total distance calculation unit 622 determines that p is equal to N (step S49: Yes), the subregion $P_m$ is positioned inside the region where all of the hemispheres corresponding to the respective receiving antennas are overlapped. Therefore, one is added to q to be q = q + 1 (step S50). Here, q represents the number of the subregions P existing inside the region where all of the hemispheres corresponding to the respective receiving antennas are overlapped.

After completion of step S50, or if p is not equal to N (step S49: No), the total distance calculation unit 622 compares the identification coefficient m of the subregion with the maximum value M of the identification coefficient m of the subregion to determine whether m is equal to M (step S51). If m is not equal to M (step S51: No), the total distance calculation unit 622 adds one to the identification coefficient m of the subregion to be m = m + 1 (step S52), and calculates, for a next subregion $P_m$, the total sum of distances $D_m$ which is the total sum of the distances between the center point of the subregion $P_m$ and the respective spherical surfaces.

Further, if m is equal to M (step S51: Yes), the total distance calculation unit 622 determines whether q representing the number of the subregions P inside the region where all of the hemispheres corresponding to the respective receiving antennas are overlapped is larger than zero (step S53).

If q is not larger than zero (step S53: No), in other words, if none of the subregions P exists in the region where all of the hemispheres corresponding to the respective receiving antennas are overlapped, the total distance calculation unit 622 executes the correction process for all of the receiving antennas to correct the distance $r_n$ in response to the dispersion of reception strength of each of the receiving antennas (step S54), and then returns to step S41 to execute the total distance calculation process for each subregion $P_1$ again.

On the other hand, if q is larger than zero (step S53: Yes), one or more of the subregions P exist in the region where all of the hemispheres corresponding to the respective receiving antennas are overlapped (step S55). Therefore, the total distance calculation unit 622 outputs each of the total sum of the distances $D_m$ calculated for each of the subregions to the position determination unit 623, thereby finishing the total distance calculation process.

Thus, in the total distance calculation process according to the present embodiment, the total sum of distances is calculated for all of the subregions $P_m$, and only in the case where it is determined that the subregion is not positioned inside the region where any of the spheres are overlapped, the correction process to correct the first distance may be executed in response to the dispersion of reception strength of all of the receiving antennas.

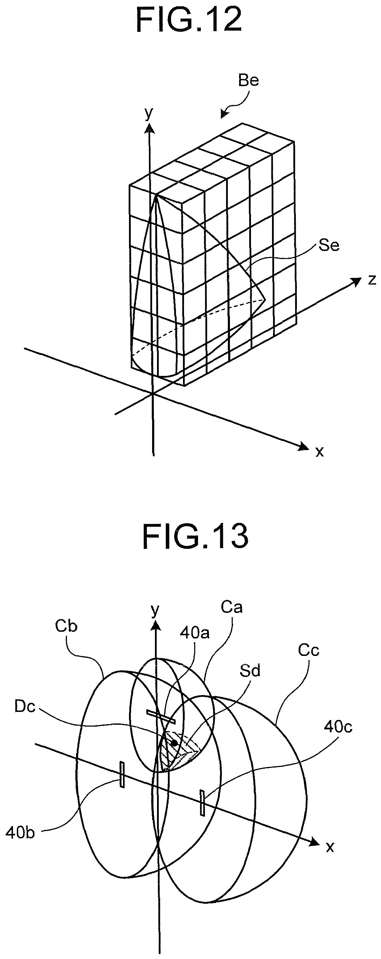

Additionally, it is determined for all of the subregions whether to exist inside the region where all of the spheres are overlapped in the total distance calculation process, but the subregion to be determined does not have to be all of the subregions and may be limited in accordance with the position of the capsule endoscope 3 previously estimated because the capsule endoscope 3 does not actually largely move in a short time. In the case where that a region Se illustrated in FIG. 12 is determined as the region where all of the spheres are overlapped in the previously executed total distance calculation process, the respective processes of the total distance calculation process may be executed for subregions of a region Be to which movable range of the capsule endoscope 3 is added centered at the region Se, to reduce the calculation amount. Of course, it is also possible to change in every total distance calculation process the size of the divided subregions in response to the region where all of the spheres obtained in the previous total distance calculation process are overlapped.

Further, according to the present embodiment, the exemplary case has been described in which the distance $r_n$ between the capsule endoscope 3 and each of the first receiving antenna 40a to the third receiving antenna 40c is obtained, and then the probable existence region of the capsule endoscope 3 is divided into the plurality of the subregions and the respective processes are executed for each of the subregions to determine the position of the capsule endoscope 3 as the total distance calculation process; however, of course, the embodiment is not limited thereto. For instance, as illustrated in FIG. 13, the distance $r_n$ between the capsule endoscope 3 and each of the first receiving antenna 40a to the third receiving antenna 40c is obtained, and then a region Sd where all of the plurality of spheres Ca to Cc is overlapped is obtained. Here, the plurality of spheres Ca to Cc centered at each of the first receiving antenna 40a to the third receiving antenna 40c has the radius of the distance $r_n$ corresponding to each of the receiving antennas. Note that, since the receiving antenna unit 4 is provided on the XY plane which is a boundary surface of the probable existence region of the capsule endoscope 3, the spheres Ca to Cc where it can be estimated that the actual capsule endoscope 3 is positioned may be considered as the hemispheres, as illustrated in FIG. 13. After that, a position Dc out of the region Sd, where the total sum of the distances from the respective spherical surfaces is minimum, is obtained by using the steepest descent method, Gauss-Newton method, and the like.

$$S(x, y, z) = \sum_{n=1}^{N} (r_n - d_n(x, y, z)) \tag{3}$$

In Expression (3), $r_n$ represents the distance between the capsule endoscope 3 and the reference position of the n-th receiving antenna, and $d_n(x, y, z)$ represents the distance between the reference position of the n-th receiving antenna and a position to be calculated. Since the region where the capsule endoscope 3 is positioned is inside each of the spheres Ca to Cc, calculation may be executed for the $d_n(x, y, z)$ in which $r_n - d_n(x, y, z)$ is equal to or larger than zero.

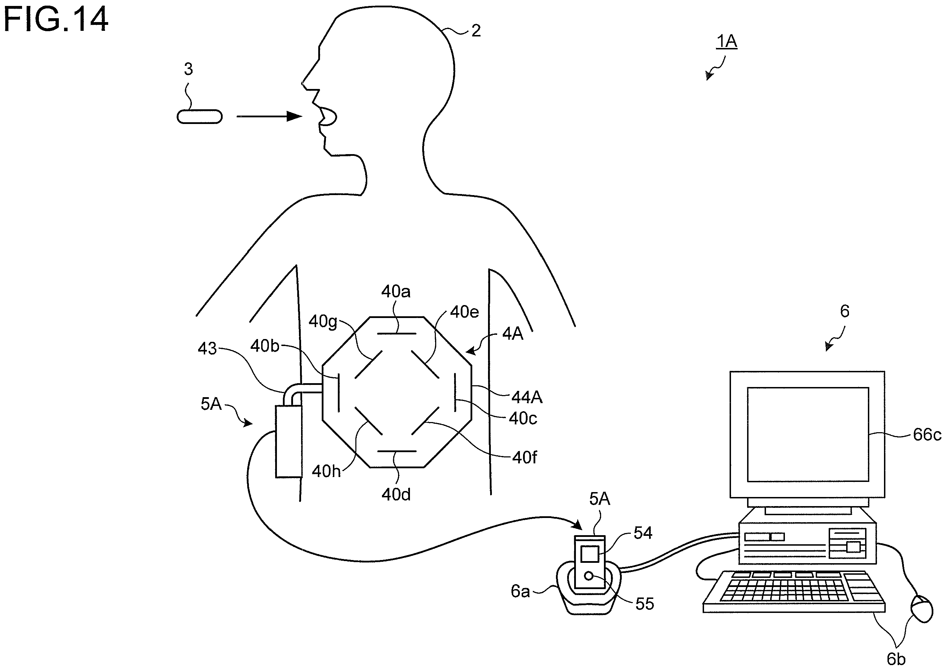

Additionally, according to the present embodiment, the exemplary case of using the three receiving antennas has been described, but not necessary to understand that the number of receiving antennas is limited to three. For example, as illustrated in a receiving antenna unit 4A connected to a receiving device 5A of a capsule endoscope system 1A in FIG. 14, a sheet 44A on which eight receiving antennas 40a to 40h are arranged may be used as well. Also, arrangement of the plurality of the receiving antennas may be suitably changed in accordance with the purpose, such as examination or diagnosis.

Further, according to the present embodiment, the information processor 6 includes the position information estimating unit 62 and the trajectory calculation unit 63, estimates the position of the capsule endoscope 3, and then calculates the trajectory. However, it is also possible to configure that the receiving device of the capsule endoscope system 1 includes an estimation unit that estimates position information and a trajectory calculation unit so that the position of the capsule endoscope 3 where the image data has been captured may be estimated.

The configuration of the receiving device in this case will be described. FIG. 15 is a block diagram illustrating another configuration of the receiving device illustrated in FIG. 1.

A receiving device 5B illustrated in FIG. 15 includes each of the above-described first receiving antenna 40a to the third receiving antenna 40c, an antenna switchover selection switching unit 49 that alternatively switches among the first receiving antenna 40a to the third receiving antenna 40c, a transceiver circuit 50 that applies a process such as demodulation to a radio signal received via any one of the first receiving antenna 40a to third receiving antenna 40c selected by the antenna switchover selection switching unit 49, a signal processing circuit 51 that executes signal processing to extract the image data and the like from the radio signal output from a transceiver circuit 50, a received electric field strength detecting unit 52 that detects received electric field strength based on the strength of the radio signal output from the transceiver circuit 50, an antenna power switchover selector 53 that alternatively switches among the first receiving antenna 40a to the third receiving antenna 40c and supplies power to any one of the first receiving antenna 40a to the third receiving antenna 40c, a display unit 54 that displays an image corresponding to the image data received from the capsule endoscope 3, an operating unit 55 for inputting operational instructions, a storage unit 56 that stores various information including the image data received from the capsule endoscope 3, an I/F unit 57 that executes transmission and reception in a mutual direction with the information processor via a cradle 6a, a power supply unit 58 that supplies the power to the respective units of the receiving device 5B, and a control unit 59 that controls operation of the receiving device 5B. Among these, the control unit 59 includes a position information estimating unit 593 that functions same as the position information estimating unit 62 illustrated in FIG. 3, and a trajectory calculation unit 597 that functions same as the trajectory calculation unit 63.

Further, the first receiving antenna 40a includes an antenna unit 41a, an active circuit 42a, and an antenna cable 43a. The antenna unit 41a is configured with, for example, an open antenna or a loop antenna, and receives the radio signal transmitted from the capsule endoscope 3. The active circuit 42a is connected to the antenna unit 41a and executes impedance matching for the antenna unit 41a, amplification or attenuation of the received radio signal and so on. The antenna cable 43a is configured with a coaxial cable, and has its one end electrically connected to the active circuit 42a and the other end electrically connected to the antenna switchover selection switching unit 49 and the antenna power switchover selector 53 of the receiving device 5, respectively. The antenna cable 43a transmits the radio signal received by the antenna unit 41a to the receiving device 5, and transfers the power supplied from the receiving device 5 to the active circuit 42a. Note that description for the second receiving antenna 40b and the third receiving antenna 40c will be omitted as these receiving antennas have the same configuration as the first receiving antenna 40a.

The antenna switchover selection switching unit 49 is configured with, for example, a mechanical switch or a semiconductor switch. The antenna switchover selection switching unit 49 is electrically connected to each of the first receiving antenna 40a to the third receiving antenna 40c via a capacitor C1. In the case where a switching signal S1 to switch among the first receiving antenna 40a to the third receiving antenna 40c for receiving the radio signal from the control unit 59 is input, the antenna switchover selection switching unit 49 selects the receiving antenna 40 instructed by the switching signal S1, and outputs, to the transceiver circuit 50, the radio signal received via the receiving antenna selected from among the first receiving antenna 40a to the third receiving antenna 40c. Note that each capacitor connected to each of the first receiving antenna 40a to the third receiving antenna 40c has the same capacitance as the capacitor C1.

The transceiver circuit 50 applies a prescribed process, such as demodulation or amplification, to the radio signal received via the receiving antenna 40 (the first receiving antenna 40a to the third receiving antenna 40c) selected by the antenna switchover selection switching unit 49, and outputs the radio signal to each of the signal processing circuit 51 and the received electric field strength detecting unit 52.

The signal processing circuit 51 extracts the image data from the radio signal input from the transceiver circuit 50, and applies a prescribed process, such as various sorts of image processing or A/D conversion processing, to the extracted image data, and outputs the image data to the control unit 59.

The received electric field strength detecting unit 52 detects the received electric field strength responsive to the strength of the radio signal input from the transceiver circuit 50, and outputs, to the control unit 59, the received electric field strength signal (RSSI: Received Signal Strength Indicator) corresponding to the detected electric field strength.

The antenna power switchover selector 53 is electrically connected to each of the first receiving antenna 40a to the third receiving antenna 40c via a coil L1. The antenna power switchover selector 53 supplies the power to one of the first receiving antenna 40a to the third receiving antenna 40c selected by the antenna switchover selection switching unit 49 via the antenna cable 43 (43a to 43c). The antenna power switchover selector 53 includes a power switchover selection switching unit 531 and an abnormality detecting unit 532. Note that the coil connected to each of the first receiving antenna 40a to the third receiving antenna 40c has the same electric properties as the coil L1.

The power switchover selection switching unit 531 is configured with, for example, a mechanical switch or a semiconductor switch. In the case where a selection signal S2 is input from the control unit 59 to select one of the first receiving antenna 40a to the third receiving antenna 40c to supply the power, the power switchover selection switching unit 531 selects one of the first receiving antenna 40a to the third receiving antenna 40c as instructed by the selection signal S2, and supplies the power only to the receiving antenna selected from among the first receiving antenna 40a to the third receiving antenna 40c.

In the case where any abnormality occurs in the first receiving antenna 40a to the third receiving antenna 40c to supply the power, the abnormality detecting unit 532 outputs, to the control unit 59, an abnormality signal indicating the occurrence of the abnormality in the first receiving antenna 40a to the third receiving antenna 40c to supply the power.

The display unit 54 is configured with a display panel formed of, for example, liquid crystal, organic EL (Electro Luminescence). The display unit 54 displays various sorts of information such as an image corresponding to image data captured by the capsule endoscope 3, an operation state of the receiving device 5, patient information of the subject 2, and an examination date and time.

The operating unit 55 may input an instruction signal, such as the instruction signal to change an imaging cycle of the capsule endoscope 3. In response to the instruction signal input from the operating unit 55, the signal processing circuit 51 transmits the instruction signal to the transceiver circuit 50, and the transceiver circuit 50 modulates the instruction signal, and then transmits the instruction signal from the first receiving antenna 40a to the third receiving antenna 40c. The instruction signal which the first receiving antenna 40a to the third receiving antenna 40c transmitted is received by an antenna 39 and demodulated by the transceiver circuit 37, and then the circuit board 36 operates to change the imaging cycle, for example, in response to the instruction signal.

The storage unit 56 is configured with a semiconductor memory such as a flash memory or a RAM (Random Access Memory) fixed inside the receiving device 5. Further, the storage unit 56 stores the image data captured by the capsule endoscope 3 and various sorts of information correlated to the image data, such as estimated position information of the capsule endoscope 3, the received electric field strength information, and identification information to identify the receiving antenna that has received the radio signal. Further, the storage unit 56 stores various programs and the like executed by the receiving device 5. Note that the storage unit 56 may be provided with a function as a recording medium interface to store information from an external unit in a recording medium such as a memory card while reading the information stored in the recording medium.

The I/F unit 57 has a function as a communication interface, and communicates bi-directionally with the information processor via the cradle 6a.

The power supply unit 58 is configured with a battery detachably attached to the receiving device 5 and a switch unit that switches between ON/OFF states. In the ON state, the power supply unit 58 supplies driving power necessary for each component in the receiving device 5, and in the OFF state the power supply unit 58 stops supplying the driving power to each component in the receiving device 5.

The control unit 59 is configured with, for example, a CPU (Central Processing Unit). The control unit 59 reads and executes the programs from the storage unit 56, and forwards the instructions, data, and the like to each component included in the receiving device 5, thereby integrally controlling the operation of the receiving device 5. The control unit 59 includes a selection controller 591, an abnormality information adding unit 592, a position information estimating unit 593, and a trajectory calculation unit 597.

The selection controller 591 selects one of the first receiving antenna 40a to the third receiving antenna 40c for receiving the radio signal transmitted from the capsule endoscope 3, and controls to supply the power only to the selected one of the first receiving antenna 40a to the third receiving antenna 40c. More specifically, at the timing of selecting the antenna, the selection controller 591 selects one receiving antenna 40 for receiving the radio signal including the image signal transmitted from the capsule endoscope 3 at the timing of receiving the image signal based on the reception strength of each of the first receiving antenna 40a to the third receiving antenna 40c detected by the received electric field strength detecting unit 52, and controls to supply the power only to the receiving antenna selected from among the first receiving antenna 40a to the third receiving antenna 40c at the timing of receiving the image signal. The selection controller 591 allows, as the timing of selecting the antenna, the received electric field strength detecting unit 52 to detect the received electric field strength of each of the receiving antennas in order to sequentially select, at a cycle of 100 msec, for example, one of the first receiving antenna 40a to the third receiving antenna 40c for receiving the radio signal including the image signal from among the first receiving antenna 40a to the third receiving antenna 40c. Then, the selection controller 591 drives the antenna switchover selection switching unit 49 to supply the power only to the receiving antenna selected from among the first receiving antenna 40a to the third receiving antenna 40c.

In the case where the abnormality detecting unit 532 detects any abnormality at any one of the first receiving antenna 40a to the third receiving antenna 40c, the abnormality information adding unit 592 adds, to the radio signal received by the receiving antenna 40, abnormality information indicating that the abnormality is occurring at any one of the first receiving antenna 40a to the third receiving antenna 40c, and output the radio signal to the storage unit 56. More specifically, the abnormality information adding unit 592 outputs the image data to the storage unit 56 after adding abnormality information (flag) to the image data for which the signal processing circuit 51 has executed the signal processing to the radio signal received by the first receiving antenna 40a to the third receiving antenna 40c. Note that the position information estimating unit 593 includes a distance calculation unit 594 that has the function same as the distance calculation unit 621 illustrated in FIG. 3, and a total distance calculation unit 595 that has the function same as the total distance calculation unit 622, and a position determination unit 596 that has the function same as the position determination unit 623.

Industrial Applicability

The receiving device and the capsule endoscope system according to the present invention is useful in detecting the position of the capsule endoscope introduced inside the subject, particularly suitable for, using an image processor, processing and diagnosing the image data captured by the capsule endoscope.

Reference Signs List

| | |
|---|---|
| 1 | CAPSULE ENDOSCOPE SYSTEM |
| 2 | SUBJECT |
| 3 | CAPSULE ENDOSCOPE |
| 4 | RECEIVING ANTENNA UNIT |
| 5 | RECEIVING DEVICE |

| | |
|---|---|
| 6 | INFORMATION PROCESSOR |
| 6a | CRADLE |
| 6b | OPERATION INOUT UNIT |
| 40a to 40h | RECEIVING ANTENNA |
| 41a to 41c | ANTENNA UNIT |
| 42a to 42c | ACTIVE CIRCUIT |
| 43a to 43c | ANTENNA CABLE |
| 44 | SHEET |
| 49 | ANTENNA SWITCHOVER SELECTION SWITCHING UNIT |
| 50 | TRANSCEIVER CIRCUIT |
| 51 | SIGNAL PROCESSING CIRCUIT |
| 52 | RECEIVED ELECTRIC FIELD STRENGTH DETECTING UNIT |
| 53 | ANTENNA POWER SWITCHOVER SELECTOR |
| 54 | DISPLAY UNIT |
| 55 | OPERATING UNIT |
| 56, 64 | STORAGE UNIT |
| 57 | I/F UNIT |
| 58 | POWER SUPPLY UNIT |
| 59, 61 | CONTROL UNIT |
| 62, 593 | POSITION INFORMATION ESTIMATING UNIT |
| 63, 597 | TRAJECTORY CALCULATION UNIT |
| 65 | INPUT UNIT |
| 66 | OUTPUT UNIT |
| 66c | DISPLAY UNIT |
| 531 | POWER SWITCHOVER SELECTION SWITCHING UNIT |
| 532 | ABNORMALITY DETECTING UNIT |
| 591 | SELECTION CONTROLLER |
| 592 | ABNORMALITY INFORMATION ADDING UNIT |
| 621, 594 | DISTANCE CALCULATION UNIT |
| 622, 595 | TOTAL DISTANCE CALCULATION UNIT |
| 623, 596 | POSITION DETERMINATION UNIT |

**Claims**

1.  A position detection apparatus for detecting a position of a capsule endoscope in a subject based on reception strength of a signal at a plurality of receiving antennas that is transmitted from the capsule endoscope configured to be introduced into the subject to move inside the subject, the position detection apparatus comprising:

    a distance calculation unit (621) configured to calculate a first distance ($r_n$) between each of the plurality of receiving antennas and the capsule endoscope based on each reception strength of the signal received by the plurality of receiving antennas;
    a total distance calculation unit (622) configured to: set a plurality of subregions obtained by dividing a region inside the subject where the capsule endoscope possibly exists; calculate a second distance ($d_{mn}$) which is a distance between a center point of each of the plurality of subregions and each of the plurality of receiving antennas; determine whether the center point of each of the plurality of subregions is positioned inside a region where at least three or more out of a plurality of spheres are overlapped, each of the plurality of spheres having a center at each of the plurality of receiving antennas and having a radius of the first distance ($r_n$), based on whether or not the second distance ($d_{mn}$) is equal to or shorter than the first distance ($r_n$); calculate, for each of the receiving antennas corresponding to the at least three or more spheres, differences between the first distance ($r_n$) and the second distance ($d_{mn}$) with respect to the center point of at least one of the plurality of subregions which has been determined to be positioned inside the region where the at least three or more spheres are overlapped, when the second distance ($d_{mn}$) is equal to or shorter than the first distance ($r_n$); and acquire a total sum of the calculated differences; and
    a position determination unit (623) configured to detect, as the position of the capsule endoscope, a position where the total sum of the calculated differences is minimum.

2.  The position detection apparatus according to claim 1, wherein the total distance calculation unit (622) is configured to correct the first distance based on dispersion of reception strength of each of the receiving antennas.

3. The position detection apparatus according to claim 2, wherein the total distance calculation unit (622) is configured to correct the first distance when the second distance which is a distance between the center point of the subregion and each of the receiving antennas is larger than the first distance for each of the receiving antennas.

4. The position detection apparatus according to claim 1, wherein the total distance calculation unit (622) is configured to determine whether the center point of each of the plurality of the subregions is positioned inside the region where all of the spheres corresponding to the plurality of the receiving antennas are overlapped.

5. The position detection apparatus according to claim 1, further comprising a trajectory calculation unit (63) configured to calculate a movement trajectory of the capsule endoscope based on the position of the capsule endoscope detected by the position determination unit.

6. The position detection apparatus according to claim 1, further comprising the plurality of the receiving antennas.

7. The position detection apparatus according to claim 6, wherein the plurality of the receiving antennas are provided on a piece of sheet.

8. A capsule endoscope system (1) comprising:

a capsule endoscope (3) configured to be introduced into a subject (2) and move inside the subject to obtain image information on an inside of the subject (2); and

the position detection apparatus according to claim 1 further comprising an image display unit (66c) configured to acquire the image information obtained by the capsule endoscope (3) and position information of the capsule endoscope (3) corresponding to the image information, and to display the acquired image information and position information.

9. The capsule endoscope system according to claim 8, wherein
the position detection apparatus further comprises a trajectory calculation unit (63) configured to calculate a movement trajectory of the capsule endoscope (3) based on the position of the capsule endoscope (3) detected by the position determination unit, and
the image display unit (66c) is configured to display the image information and also display the movement trajectory of the capsule endoscope (3) inside the subject (2) calculated by the trajectory calculation unit (63).

10. A position detecting program for a capsule endoscope causing a position detection apparatus for detecting a position of the capsule endoscope in a subject based on reception strength of a signal at a plurality of receiving antennas that is transmitted from the capsule endoscope configured to be introduced into the subject to move inside the subject, to execute:

a distance calculation step of calculating a first distance ($r_n$) between each of the plurality of receiving antennas and the capsule endoscope based on each reception strength of the signal received by the plurality of receiving antennas;
setting a plurality of subregions obtained by dividing a region inside the subject where the capsule endoscope possibly exists;
calculating a second distance ($d_{mn}$) which is a distance between a center point of each of the plurality of subregions and each of the plurality of receiving antennas;
determining whether the center point of each of the plurality of subregions is positioned inside a region where at least three or more out of a plurality of spheres are overlapped, each of the plurality of spheres having a center at each of the plurality of receiving antennas and having a radius of the first distance ($r_n$), based on whether or not the second distance ($d_{mn}$) is equal to or shorter than the first distance ($r_n$);
calculating, for each of the receiving antennas corresponding to the at least three or more spheres, differences between the first distance ($r_n$) and the second distance ($d_{mn}$) with respect to the center point of at least one of the plurality of subregions which has been determined to be positioned inside the region where the at least three or more spheres are overlapped, when the second distance ($d_{mn}$) is equal to or shorter than the first distance ($r_n$);
acquiring a total sum of the calculated differences; and

a detecting step of detecting, as the position of the capsule endoscope, a position where the total sum of the calculated differences is minimum.

**Patentansprüche**

1. Positionserfassungsgerät zum Erfassen einer Position eines Kapselendoskops in einem Subjekt auf der Basis einer Empfangsstärke eines Signals an einer Mehrzahl von Empfangsantennen, das von dem Kapselendoskop übertragen wird, welches dazu eingerichtet ist, in das Subjekt eingeführt zu werden, um sich im Inneren des Subjekts zu bewegen, wobei das Positionserfassungsgerät umfasst:

   eine Entfernungsberechnungseinheit (621), die dazu eingerichtet ist, eine erste Entfernung ($r_n$) zwischen jeder der Mehrzahl von Empfangsantennen und dem Kapselendoskop auf der Basis jeder Empfangsstärke des von der Mehrzahl von Empfangsantennen empfangenen Signals zu berechnen;
   eine Gesamtentfernungsberechnungseinheit (622), die dazu eingerichtet ist: eine Mehrzahl von Unterbereichen festzulegen, die durch Teilen eines Bereichs im Inneren des Subjekts, in dem sich das Kapselendoskop möglicherweise befindet, erhalten werden; eine erste Entfernung ($d_{mn}$) zu berechnen, die eine Entfernung zwischen einem Mittelpunkt eines jeden der Mehrzahl von Unterbereichen und jeder der Mehrzahl von Empfangsantennen ist; zu bestimmen, ob der Mittelpunkt eines jeden der Mehrzahl von Unterbereichen innerhalb eines Bereichs angeordnet ist, in dem mindestens drei oder mehr einer Mehrzahl von Sphären überlappen, wobei jede der Mehrzahl von Sphären einen Mittelpunkt an jeder der Mehrzahl von Empfangsantennen hat und einen Radius der ersten Entfernung ($r_n$) hat, basierend darauf, ob die zweite Entfernung ($d_{mn}$) gleich der oder kürzer als die erste Entfernung ($r_n$) ist; für jede der Empfangsantennen, die den mindestens drei oder mehr Sphären entspricht, Unterschiede zwischen der ersten Entfernung ($r_n$) und der zweiten Entfernung ($d_{mn}$) bezüglich des Mittelpunkts mindestens eines der Mehrzahl von Unterbereichen zu berechnen, für den bestimmt worden ist, dass er innerhalb des Bereichs angeordnet ist, in dem die mindestens drei oder mehr Sphären überlappen, wenn die zweite Entfernung ($d_{mn}$) gleich der oder kürzer als die erste Entfernung ($r_n$) ist; und eine Gesamtsumme der berechneten Unterschiede zu erfassen; und
   eine Positionsbestimmungseinheit (623), die dazu eingerichtet ist, als die Position des Kapselendoskops eine Position zu erfassen, an der die Gesamtsumme der berechneten Unterschiede minimal ist.

2. Positionserfassungsgerät nach Anspruch 1, bei dem die Gesamtentfernungsberechnungseinheit (622) dazu eingerichtet ist, die erste Entfernung auf der Basis einer Streuung einer Empfangsstärke einer jeden der Empfangsantennen zu korrigieren.

3. Positionserfassungsgerät nach Anspruch 2, bei dem die Gesamtentfernungsberechnungseinheit (622) dazu eingerichtet ist, die erste Entfernung zu korrigieren, wenn die zweite Entfernung, die eine Entfernung zwischen dem Mittelpunkt des Unterbereichs und jeder der Empfangsantennen ist, für jede der Empfangsantennen größer ist als die erste Entfernung.

4. Positionserfassungsgerät nach Anspruch 1, bei dem die Gesamtentfernungsberechnungseinheit (622) dazu eingerichtet ist, zu bestimmen, ob der Mittelpunkt eines jeden der Mehrzahl der Unterbereiche innerhalb des Bereichs angeordnet ist, in dem alle Sphären, die der Mehrzahl der Empfangsantennen entsprechen, überlappen.

5. Positionserfassungsgerät nach Anspruch 1, das ferner eine Bahnberechnungseinheit (63) umfasst, die dazu eingerichtet ist, eine Bewegungsbahn des Kapselendoskops auf der Basis der von der Positionsbestimmungseinheit erfassten Position des Kapselendoskops zu berechnen.

6. Positionserfassungsgerät nach Anspruch 1, das ferner die Mehrzahl von Empfangsantennen umfasst.

7. Positionserfassungsgerät nach Anspruch 6, bei dem die Mehrzahl der Empfangsantennen auf einem Teil einer Folie vorgesehen ist.

8. Kapselendoskopsystem (1), das umfasst:

   ein Kapselendoskop (3), das dazu eingerichtet ist, in ein Subjekt (2) eingeführt zu werden und sich im Inneren des Subjekts zu bewegen, um eine Bildinformation über ein Inneres des Subjekts (2) zu erhalten; und

das Positionserfassungsgerät nach Anspruch 1, das ferner eine Bildanzeigeeinheit (66c) umfasst, die dazu eingerichtet ist, die von dem Kapselendoskop (3) erhaltene Bildinformation und eine der Bildinformation entsprechende Positionsinformation des Kapselendoskops (3) zu erfassen, und die erfasste Bildinformation und Positionsinformation anzuzeigen.

**9.** Kapselendoskopsystem nach Anspruch 8, bei dem
das Positionserfassungsgerät ferner eine Bahnberechnungseinheit (63) umfasst, die dazu eingerichtet ist, eine Bewegungsbahn des Kapselendoskops auf der Basis der von der Positionsbestimmungseinheit erfassten Position des Kapselendoskops zu berechnen, und
die Bildanzeigeeinheit (66c) dazu eingerichtet ist, die Bildinformation anzuzeigen und darüber hinaus die von der Bahnberechnungseinheit (36) berechnete Bewegungsbahn des Kapselendoskops (3) im Inneren des Subjekts (2) anzuzeigen.

**10.** Positionserfassungsprogramm für ein Kapselendoskop, das ein Positionserfassungsgerät zum Erfassen einer Position eines Kapselendoskops in einem Subjekt veranlasst, auf der Basis einer Empfangsstärke eines Signals an einer Mehrzahl von Empfangsantennen, das von dem Kapselendoskop übertragen wird, welches dazu eingerichtet ist, in das Subjekt eingeführt zu werden, um sich im Inneren des Subjekts zu bewegen, auszuführen:

einen Entfernungsberechnungsschritt zum Berechnen einer ersten Entfernung ($r_n$) zwischen jeder der Mehrzahl von Empfangsantennen und dem Kapselendoskop auf der Basis jeder Empfangsstärke des von der Mehrzahl von Empfangsantennen empfangenen Signals;
Festlegen einer Mehrzahl von Unterbereichen, die durch Teilen eines Bereichs im Inneren des Subjekts, in dem sich das Kapselendoskop möglicherweise befindet, erhalten werden;
Berechnen einer ersten Entfernung ($d_{mn}$), die eine Entfernung zwischen einem Mittelpunkt eines jeden der Mehrzahl von Unterbereichen und jeder der Mehrzahl von Empfangsantennen ist;
Bestimmen, ob der Mittelpunkt eines jeden der Mehrzahl von Unterbereichen innerhalb eines Bereichs angeordnet ist, in dem mindestens drei oder mehr einer Mehrzahl von Sphären überlappen, wobei jede der Mehrzahl von Sphären einen Mittelpunkt an jeder der Mehrzahl von Empfangsantennen hat und einen Radius der ersten Entfernung ($r_n$) hat, basierend darauf, ob die zweite Entfernung ($d_{mn}$) gleich der oder kürzer als die erste Entfernung ($r_n$) ist;
für jede der Empfangsantennen, die den mindestens drei oder mehr Sphären entspricht Berechnen, Berechnen von Unterschieden zwischen der ersten Entfernung ($r_n$) und der zweiten Entfernung ($d_{mn}$) bezüglich des Mittelpunkts mindestens eines der Mehrzahl von Unterbereichen, für den bestimmt worden ist, dass er innerhalb des Bereichs angeordnet ist, in dem die mindestens drei oder mehr Sphären überlappen, wenn die zweite Entfernung ($d_{mn}$) gleich der oder kürzer als die erste Entfernung ($r_n$) ist;
Erfassen einer Gesamtsumme der berechneten Unterschiede; und
einen Positionsbestimmungsschritt zum Erfassen einer Position, an der die Gesamtsumme der berechneten Unterschiede minimal ist, als die Position des Kapselendoskops.

**Revendications**

**1.** Appareil de détection de position destiné à détecter une position d'un endoscope de type capsule dans un sujet sur la base d'une force de réception d'un signal au niveau d'une pluralité d'antennes de réception qui est transmis depuis l'endoscope de type capsule configuré pour être introduit dans le sujet pour se déplacer à l'intérieur du sujet, l'appareil de détection de position comprenant :

une unité (621) de calcul de distance configurée pour calculer une première distance ($r_n$) entre chacune parmi la pluralité d'antennes de réception et l'endoscope de type capsule sur la base de chaque force de réception du signal reçu par la pluralité d'antennes de réception ;
une unité (622) de calcul de distance totale configurée pour : établir une pluralité de sous-régions obtenues en divisant une région à l'intérieur du sujet où l'endoscope de type capsule se trouve probablement ; calculer une deuxième distance ($d_{mn}$) qui est une distance entre un point central de chacune parmi la pluralité de sous-régions et chacune parmi la pluralité d'antennes de réception ; déterminer si le point central de chacune parmi la pluralité de sous-régions est positionné à l'intérieur d'une région où au moins trois ou plus parmi une pluralité de sphères se chevauchent, chacune parmi la pluralité de sphères comportant un centre au niveau de chacune parmi la pluralité d'antennes de réception et présentant un rayon de la première distance ($r_n$) selon si oui ou non la deuxième distance ($d_{mn}$) est égale à la première distance ($r_n$) ou plus courte que celle-ci ; calculer, pour chacune des antennes de réception correspondant aux au moins trois sphères ou plus, des différences entre la première distance ($r_n$) et la deuxième distance ($d_{mn}$) par rapport au point central d'au moins l'une parmi la pluralité de sous-régions qui a été déterminé comme étant positionné à l'intérieur de la région où les au moins trois sphères ou plus se chevauchent, lorsque la deuxième distance ($d_{mn}$) est égale à la première distance ($r_n$) ou plus courte que celle-ci ; et acquérir une somme totale des différences calculées ; et
une unité (623) de détermination de position configurée pour détecter, en tant que position de l'endoscope de type capsule, une position où la somme totale des différences calculées est minimale.

**2.** Appareil de détection de position selon la revendication 1, dans lequel l'unité (622) de calcul de distance totale est configurée pour corriger la première distance sur la base de la dispersion de la force de réception de chacune des antennes de réception.

**3.** Appareil de détection de position selon la revendication 2, dans lequel l'unité (622) de calcul de distance totale est configurée pour corriger la première distance lorsque la deuxième distance qui est une distance entre le point central de la sous-région et chacune des antennes de réception est supérieure à la première distance pour chacune des antennes de réception.

**4.** Appareil de détection de position selon la revendication 1, dans lequel l'unité (622) de calcul de distance est configurée pour déterminer si le point central de chacune parmi la pluralité de sous-régions est positionné à l'intérieur de la région où toutes les sphères correspondant à la pluralité d'antennes de réception se chevauchent.

**5.** Appareil de détection de position selon la revendication 1, comprenant en outre une unité (63) de calcul de trajectoire configurée pour calculer une trajectoire de déplacement de l'endoscope de type capsule sur la base de la position de l'endoscope de type capsule détectée par l'unité de détermination de position.

**6.** Appareil de détection de position selon la revendication 1, comprenant en outre la pluralité d'antennes de réception.

**7.** Appareil de détection de position selon la revendication 6, dans lequel la pluralité d'antennes de réception sont prévues sur un morceau de feuille.

**8.** Système (1) d'endoscope de type capsule comprenant :

un endoscope de type capsule (3) configuré pour être introduit dans un sujet (2) et se déplacer à l'intérieur du sujet pour obtenir des informations d'image sur un intérieur du sujet (2) ; et

l'appareil de détection de position selon la revendication 1, comprenant en outre une unité (66c) d'affichage d'image configurée pour acquérir les informations d'image obtenues par l'endoscope de type capsule (3) et des informations de position de l'endoscope de type capsule (3) correspondant aux informations d'image, et pour afficher les informations d'image obtenues et des informations de position.

**9.** Système d'endoscope de type capsule selon la revendication 8, dans lequel
l'appareil de détection de position comprend en outre une unité (63) de calcul de trajectoire configurée pour calculer une trajectoire de déplacement de l'endoscope de type capsule (3) sur la base de la position de l'endoscope de type capsule (3) détectée par l'unité de détermination de position, et
l'unité (66c) d'affichage d'image est configurée pour afficher les informations d'image et également afficher la trajectoire de déplacement de l'endoscope de type capsule (3) à l'intérieur du sujet (2) calculée par l'unité (63) de calcul de trajectoire.

**10.** Programme de détection de position pour un endoscope de type capsule amenant un appareil de détection de position à détecter une position de l'endoscope de type capsule dans un sujet sur la base d'une force de réception d'un signal au niveau d'une pluralité d'antennes de réception qui est transmis depuis l'endoscope de type capsule configuré pour être introduit dans le sujet pour se déplacer à l'intérieur du sujet, pour exécuter :

une étape de calcul de distance consistant à calculer une première distance ($r_n$) entre chacune parmi la pluralité d'antennes de réception et l'endoscope de type capsule sur la base de chaque force de réception du signal reçu par la pluralité d'antennes de réception ;
une étape consistant à établir une pluralité de sous-régions obtenues en divisant une région à l'intérieur du sujet où l'endoscope de type capsule se trouve probablement ;

une étape consistant à calculer une deuxième distance ($d_{mn}$) qui est une distance entre un point central de chacune parmi la pluralité de sous-régions et chacune parmi la pluralité d'antennes de réception ;

une étape consistant à déterminer si le point central de chacune parmi la pluralité de sous-régions est positionné à l'intérieur d'une région où au moins trois ou plus parmi une pluralité de sphères se chevauchent, chacune parmi la pluralité de sphères comportant un centre au niveau de chacune parmi la pluralité d'antennes de réception et présentant un rayon de la première distance ($r_n$), selon si oui ou non la deuxième distance ($d_{mn}$) est égale à la première distance ($r_n$) ou plus courte que celle-ci ;

une étape consistant à calculer, pour chacune des antennes de réception correspondant aux au moins trois sphères ou plus, des différences entre la première distance ($r_n$) et la deuxième distance ($d_{mn}$) par rapport au point central d'au moins l'une parmi la pluralité de sous-régions qui a été déterminé comme étant positionné à l'intérieur de la région où au moins trois sphères ou plus se chevauchent, lorsque la deuxième distance ($d_{mn}$) est égale à la première distance ($r_n$) ou plus courte que celle-ci ;

une étape consistant à acquérir une somme totale des différences calculées ; et

une étape de détection consistant à détecter, en tant que position de l'endoscope de type capsule, une position où la somme totale des différences calculées est minimale.

FIG.1

# FIG.2

35 34 38 30a (30) 3

33

32

30b
(30)

36 37 39

# FIG.3

6

POSITION
INFORMATION
ESTIMATING UNIT
62

DISTANCE
CALCULATION
UNIT
621

STORAGE UNIT
64

TOTAL DISTANCE
CALCULATION
UNIT
622

61

POSITION
DETERMINATION
UNIT
623

CONTROL
UNIT

INPUT UNIT
65

OUTPUT UNIT
66

66c

DISPLAY
UNIT

TRAJECTORY
CALCULATION UNIT
63

# FIG.4

```
            START

ACQUIRE SIGNAL STRENGTH AT          S1
RESPECTIVE RECEIVING ANTENNAS

POSITION DETERMINING PROCESS        S2

MOVEMENT TRAJECTORY                 S3
CALCULATION PROCESS

             END
```

# FIG.5

```
    POSITION DETERMINING
         PROCESS

ACQUIRE DISTANCE $r_n$ BETWEEN      S11
EACH OF RECEIVING ANTENNAS AND
      CAPSULE ENDOSCOPE

TOTAL DISTANCE CALCULATION          S12
         PROCESS

ACQUIRE POSITION AT WHICH TOTAL     S13
   SUM OF DISTANCES IS MINIMUM

DETERMINE ACQUIRED POSITION AS      S14
POSITION OF CAPSULE ENDOSCOPE

            RETURN
```

# FIG.6A

# FIG.6B

# FIG.7

# FIG.8

TOTAL DISTANCE
CALCULATION PROCESS

$m=1$ — S21

$n=1$ — S22

ACQUIRE DISTANCE $d_{mn}$ BETWEEN n-th RECEIVING ANTENNA AND CENTER POINT OF SUBREGION $P_m$ — S23

S24 — $d_{mn} \leq r_n$?

YES

S28 — $n=N$?

NO → S29 — $n=n+1$

YES

S30 — CALCULATE TOTAL SUM OF DISTANCES $D_m$

NO (from S24) → S25 — HAS COR-RECTION BEEN DONE?

YES → S27 — CENTER POINT OF SUBREGION $P_m$ IS NOT ADOPTED

NO → S26 — CORRECTION PROCESS

S31 — $m=M$?

NO → S32 — $m=m+1$

YES

OUTPUT EACH TOTAL SUM OF DISTANCES $D_m$ — S33

RETURN

EP 2 737 842 B1

# FIG.9

# FIG.10

# FIG.11

```
          ( TOTAL DISTANCE
            CALCULATION PROCESS )
                    │
                    ▼
        ┌───────────────────────┐
        │       m=1, q=0         │──── S41
        └───────────────────────┘
                    │
                    ▼
        ┌───────────────────────┐
        │       n=1, p=0         │──── S42
        └───────────────────────┘
                    │
                    ▼
        ┌───────────────────────────┐
        │ ACQUIRE DISTANCE dmn       │
        │ BETWEEN n-th RECEIVING     │──── S43
        │ ANTENNA AND CENTER POINT   │
        │ OF SUBREGION Pm            │
        └───────────────────────────┘
                    │
                    ▼
   NO        ╱──── S44 ───╲
  ◄──────────    dmn ≤ rn?  
            ╲────────────╱
                    │ YES
                    ▼
        ┌───────────────────────┐
        │        p=p+1          │──── S45
        └───────────────────────┘
                    │
                    ▼
            ╱──── S46 ───╲        NO
            ──    n=N?    ──────────────►  ┌──────────────┐
            ╲────────────╱                 │    n=n+1     │── S47
                    │ YES                   └──────────────┘
                    ▼ S48
        ┌───────────────────────┐
        │ CALCULATE TOTAL SUM OF│
        │     DISTANCES Dm       │
        └───────────────────────┘
                    │
                    ▼
   NO        ╱──── S49 ───╲
  ◄──────────    p=N?     
            ╲────────────╱
                    │ YES
                    ▼
        ┌───────────────────────┐
        │        q=q+1          │──── S50
        └───────────────────────┘
                    │
                    ▼
            ╱──── S51 ───╲        NO
            ──    m=M?    ──────────────►  ┌──────────────┐
            ╲────────────╱                 │    m=m+1     │── S52
                    │ YES                   └──────────────┘
                    ▼
            ╱──── S53 ───╲        NO
            ──    q>0?    ──────────────►  ┌──────────────────────┐
            ╲────────────╱                 │  CORRECTION PROCESS  │── S54
                    │ YES                   └──────────────────────┘
                    ▼ S55
        ┌───────────────────────┐
        │ OUTPUT EACH TOTAL SUM │
        │  OF DISTANCES Dm       │
        └───────────────────────┘
                    │
                    ▼
              ( RETURN )
```

# FIG.12

# FIG.13

FIG.14

# FIG.15

EP 2 737 842 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007000608 A
- JP 2006271987 A
- EP 1260176 A1
- US 2008312501 A1